# EUROPEAN PATENT SPECIFICATION

(11) **EP 2 078 750 B1**
(45) Date of publication and mention of the grant of the patent: **28.09.2016**
(21) Application number: 08000297.5
(22) Date of filing: 09.01.2008
(51) Int. Cl.: C12N 15/10, G01N 33/542, C12Q 1/68

(54) **A fluorescent two-hybrid (F2H) assay for direct visualization of protein interactions in living cells**
Fluoreszenter Zwei-Hybrid(F2H)-Assay zur direkten Sichtbarmachung von Proteininteraktionen in lebenden Zellen
Analyse fluorescente à deux hybrides (F2H) pour virtualisation directe des interactions de protéines dans des cellules vivantes

(43) Date of publication of application: 15.07.2009
(73) Proprietor: Ludwig-Maximilians-Universität München, 80539 München (DE)
(72) Inventor: Rothbauer, Ulrich, 80796 München (DE); Leonhardt, Heinrich, 80331 München (DE); Zolghadr, Kourosh, 80796 München (DE); Mortusewicz, Oliver, 80469 München (DE)
(74) Representative: Vossius & Partner Patentanwälte Rechtsanwälte mbB

(56) References cited:
- EP-A- 1 785 434
- WO-A-01/36671
- WO-A-02/086450
- WO-A-2005/030989
- WO-A-2006/099486
- US-A1- 2002 177 217
- US-A1- 2006 051 739
- TUMBAR T ET AL: "Large-scale chromatin unfolding and remodeling induced by VP16 acidic activation domain." THE JOURNAL OF CELL BIOLOGY 28 JUN 1999, vol. 145, no. 7, 28 June 1999 (1999-06-28), pages 1341-1354, XP002474322 ISSN: 0021-9525

## Description

The present invention relates to an in vitro method for detecting protein-protein interactions comprising: (a) expressing in a eukaryotic cell a first fusion protein comprising (i) a (poly)peptide that, when expressed in a cell, accumulates at distinct sites in the nucleus of the cell; and (ii) a (poly)peptide specifically binding to GFP; (b) expressing in the same cell a second fusion protein comprising (i) GFP; and (ii) a bait (poly)peptide; (c) expressing in the same cell a third fusion protein comprising (i) a fluorescent (poly)peptide, the excitation and/or emission wavelength of which differs from that of GFP; and (ii) a prey (poly)peptide; and (d) detecting the fluorescence emission of the fluorescent parts of the second and the third fusion protein in the cell upon excitation, wherein a co-localization of the fluorescence emission of both fusion proteins in the cell nucleus is indicative of an interaction of the bait and the prey (poly)peptide. The invention also relates to an in vitro method for detecting protein-protein interactions comprising: (a) expressing in a eukaryotic cell a first fusion protein comprising (i) a fluorescent (poly)peptide; (ii) a (poly)peptide that, when expressed in a cell, accumulates at distinct sites in the nucleus of the cell; and (iii) a bait (poly)peptide; (b) expressing in the same cell a second fusion protein comprising (i) a fluorescent (poly)peptide, the excitation and/or emission wavelength of which differs from that of the fluorescent (poly)peptide comprised in said first fusion protein; and (ii) a prey (poly)peptide and (c) detecting the fluorescence emission of the fluorescent parts of the first and the second fusion protein in the cell upon excitation, wherein a co-localization of the fluorescence emission of both fusion proteins in the cell nucleus is indicative of an interaction of the bait and the prey (poly)peptide. Furthermore, the present invention relates to methods for identifying a compound modulating the interaction of two (poly)peptides and methods of determining the relative strength of the interaction of two proteins with a third protein.

In this specification, a number of documents are cited.

After sequencing the human genome, the next challenge is now to analyze the complex protein-networks underlying cellular functions. In the last decade, a wide variety of methods to study protein-protein interactions ranging from biochemical to genetic or cell-based approaches have been developed. Biochemical methods such as affinity purification or co-immunoprecipitation (Co-IP) allow the detection of protein complexes *in vitro.* Genetic methods, such as the yeast two-hybrid (Y2H) system, enable efficient high-throughput screening of interactions within the cellular environment. The analysis of mammalian protein-protein interactions in yeast may, however, suffer from the absence or insufficient conservation of cellular factors modulating protein-protein interactions, e.g. through posttranslational modifications (Parrish et al., 2006). Furthermore, this method is laborious and error-prone.
In recent years new fluorescence-based methods for in-cell visualization of protein-protein-interactions have been introduced. Two established techniques, fluorescence resonance energy transfer (FRET) (Miyawaki, 2003; Sekar and Periasamy, 2003) and bimolecular fluorescence complementation (BiFC) (Kerppola, 2006), are based on the expression of fluorescently labeled proteins or fragments thereof. However, FRET requires costly instrumentation and advanced technical expertise, while BiFC is based on the irreversible complementation and slow maturation of fluorophores which does not allow real-time detection of protein-protein interactions (Kerppola, 2006).

Tumbar *et al.* 1999 describes a nucleic acid molecule encoding a fusion protein comprising a fluorescent protein, an NLS and a bait protein (the acidic activation domain of VP16) but does not disclose the stable expression of the construct in cells.

US 2006/051739 describes methods of visualizing and interfering with chromosomal tethering and segregation of extrachromosomal molecules, however neither a fluorescent protein nor a bait protein is present in the fusion proteins employed. Also disclosed are fusion constructs comprising GFP or YFP, the lac repressor Lacl and a nuclear localization signal but the fusion construct lacks a bait protein.

WO 2005/030989 describes methods of detecting proteins using aptamers. Specifically, WO 2005/030989 discloses nucleic acids comprising fusions of the FLAG epitope with the lac repressor Lacl or the tet repressor TetR. Further disclosed are fusions comprising nucleic acids encoding FLAG, Lacl, S35, HA, TetR and PML. Nucleic acids encoding a fusion protein comprising a fluorescent protein and a protein accumulating at distinct sites in the nucleus of the cell and a bait protein are not described.

WO 02/086450 describes a targeting domain that is used to immobilize the bait-(poly)peptide at a subcellular localisation. Importantly, the bait protein is not fused to a fluorescent protein but the "bait fusion" consists of a ligand receptor of the bait fusion that may be ecdysone as well as a further compound. Thus, in WO 02/086450 the bait protein is not detectably labelled, and an indirect assessment of the localization by means of a detectable label fused to the prey protein is required, which may lead to an enhanced frequency of false positive results since the detectable prey protein may aggregate or bind to cellular structures via unspecific interactions (including hydrophilic or hydrophobic interactions) not provided by the bait protein.

All these methods have inherent shortcomings and are typically combined to obtain more reliable results.

Thus, there is a need for improved methods of detecting protein-protein interactions.

The solution to said technical problem is achieved by providing the embodiments characterized in the claims.

Accordingly, in a first aspect the present invention relates to an in vitro method for detecting protein-protein interactions comprising: (a) expressing in a eukaryotic cell a first fusion protein comprising (i) a (poly)peptide that, when expressed in a cell, accumulates at distinct sites in the nucleus of the cell; and (ii) a (poly)peptide specifically binding to GFP; (b) expressing in the same cell a second fusion protein comprising (i) GFP; and (ii) a bait (poly)peptide; (c) expressing in the same cell a third fusion protein comprising (i) a fluorescent (poly)peptide, the excitation and/or emission wavelength of which differs from that of GFP; and (ii) a prey (poly)peptide; and (d) detecting the fluorescence emission of the fluorescent parts of the second and the third fusion protein in the cell upon excitation, wherein a co-localization of the fluorescence emission of both fusion proteins in the cell nucleus is indicative of an interaction of the bait and the prey (poly)peptide.

The term "protein-protein interactions" refers to the specific interaction of two or more proteinaceous compounds, i.e. poly(peptides) or proteins. Specific interaction is characterized by a minimum binding strength or affinity. Binding affinities for specific interactions generally reach from the pM to the mM range and also largely depend on the chemical environment, e.g. the pH value, the ionic strength, the presence of co-factors etc. In the context of the present invention, the term particularly refers to protein-protein interactions occurring under physiological conditions, i.e. in a cell.

The term "expressing in a eukaryotic cell" relates to the transcription and translation of the fusion proteins of the invention using appropriate expression control elements that function in the chosen cell. In this manner, the binding properties of individual fusion proteins may be tested in cellular expression systems. To this end, a nucleic acid molecule encoding a fusion protein may be cloned into a suitable expression vector, the composition of which generally depends on the expression system. For the present invention, the expression system is eukaryotic, preferably mammalian. A typical mammalian expression vector contains a promoter element, which mediates the initiation of transcription of mRNA, the protein coding sequence, and signals required for the termination of transcription and polyadenylation of the transcript. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Possible examples for regulatory elements ensuring the initiation of transcription comprise the cytomegalovirus (CMV) promoter, RSV-promoter (Rous sarcoma virus), the lacZ promoter, the gal10 promoter, human elongation factor 1a-promoter, CMV enhancer, CaM-kinase promoter, the Autographa californica multiple nuclear polyhedrosis virus (AcMNPV) polyhedral promoter or the SV40-enhancer. Examples for transcription termination signals are the SV40-poly-A site or the tk-poly-A site or the SV40, lacZ and AcMNPV polyhedral polyadenylation signals, downstream of the polynucleotide.Moreover, elements such as origin of replication, drug resistance genes, regulators (as part of an inducible promoter) or internal ribosomal entry sites (IRES) may also be included.
Suitable expression vectors for Drosophila are those belonging to the pMT DES system (Invitrogen) using the drosophila metallothionein (MT) promoter (Bunch et al., 1988) or pAC5.1 using the drosophila actin 5C promoter. A vector using the GAL4-inducible USA promoter is pUAST. Yeast vectors are the pYEp vector (using a Gal10 promoter, pYX142 (single copy vector) or pYX232 (2µ plasmid using the TPI triosephosphat isomerase promoter (both Novagen).

Mammalian host cells that could be used include but are not restricted to human Hela, 293, H9, SH-EP1 and Jurkat cells, mouse NIH3T3 and C2C12 cells, Cos 1, Cos 7 and CV1, quail QC1-3 cells, mouse L cells, Syrian golden baby hamster kidney (BHK) cells and Chinese hamster ovary (CHO) cells. Alternatively, the fusion proteins can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid molecule can also be amplified in the cell to express large amounts of the encoded fusion protein. The DHFR (dihydrofolate reductase) marker is useful to develop cell lines that carry several hundred or even several thousand copies of the gene of interest. Another useful selection marker is the enzyme glutamine synthase (GS) (Bebbington et al., 1992; Murphy et al., 1991). Using these markers, the mammalian cells are grown in selective medium and the cells with the highest resistance are selected. Appropriate culture media and conditions for the above-described host cells are known in the art.

The term "fusion protein" refers to chimeric proteins consisting of sequences derived from at least two different proteins or (poly)peptides. According to the teaching of the present invention, in exemplary fusion proteins, a bait (poly)peptide is fused to a fluorescent (poly)peptide and to a (poly)peptide that, when expressed in a cell, accumulates at distinct sites in the nucleus of the cell. Alternatively, a prey (poly)peptide is fused to a fluorescent (poly)peptide. Fusion may be performed by any technique known to the skilled person, as long as it results in the in frame fusion of the nucleic acid molecules encoding the components of the fusion proteins of the invention. Fusion of the components may be effected in any order. Conventionally, the generation of a fusion protein from two or more separate (poly)peptides or domains is based on the "two-sided splicing by overlap extension" described in (Horton et al., 1989). The fragments coding for the single (poly)peptides are generated in two separate primary PCR reactions. The inner primers for the primary PCR reactions contain a significant, approximately 20 bp, complementary region that allows the fusion of the two domain fragments in the second PCR. Alternatively, the coding regions may be fused by making use of restriction sites which may either be naturally occurring or be introduced by recombinant DNA technology.

The term "(poly)peptide" as used herein describes a group of molecules which comprises the group of peptides, consisting of up to 30 amino acids, as well as the group of polypeptides, consisting of more than 30 amino acids. Also in line with the definition the term "(poly)peptide" describes fragments of proteins. (Poly)peptides may further form dimers, trimers and higher oligomers, i.e. consisting of more than one (poly)peptide molecule. (Poly)peptide molecules forming such dimers, trimers etc. may be identical or non-identical. The corresponding higher order structures are, consequently, termed homo- or heterodimers, homo- or heterotrimers etc. The terms "(poly)peptide" and "protein" also refer to naturally modified (poly)peptides/proteins wherein the modification is effected e.g. by glycosylation, acetylation, phosphorylation and the like. Such modifications are well known in the art.

The term "fluorescent (poly)peptide" or "fluorescent protein" refers to (poly)peptides emitting fluorescent light upon excitation at a specific wavelength. A variety of fluorescent proteins can be used in the present invention. One group of such fluorescent proteins includes Green Fluorescent Protein isolated from *Aequorea victoria* (GFP), as well as a number of GFP variants, such as cyan fluorescent protein, blue fluorescent protein, yellow fluorescent protein, etc. (Zhang et al., 2002; Zimmer, 2002). Typically, these variants share about 80%, or greater sequence identity with the amino acid sequence of SEQ ID No: 1 or the nucleic acid sequence of SEQ ID NO: 2, respectively. Color-shift GFP mutants have emission colors blue to yellow-green, increased brightness, and photostability (Tsien, 1998). One such GFP mutant, termed the Enhanced Yellow Fluorescent Protein, displays an emission maximum at 529 nm. Additional GFP-based variants having modified excitation and emission spectra (Tsien et al., U.S. Patent Appn. 200201231 13A1), enhanced fluorescence intensity and thermal tolerance (Thastrup et al., U.S. Patent Appn. 20020107362A1; Bjorn et al., U.S. Patent Appn. 20020177189A1), and chromophore formation under reduced oxygen levels (Fisher, U.S. Patent No. 6,414,119) have also been described.

Another group of fluorescent proteins includes the fluorescent proteins isolated from anthozoans, including without limitation the red fluorescent protein isolated from *Discosoma* species of coral , DsRed (Matz et al., 1999), e.g., the amino acid sequence of SEQ ID NO: 3 or the nucleic acid sequence of SEQ ID NO: 4, respectively (see, e.g., accession number AF168419 version AF168492). DsRed and the other anthozoan fluorescent proteins share only about 26-30% amino acid sequence identity to the wild-type GFP from *Aequorea victoria,* yet all the crucial motifs are conserved, indicating the formation of the 11 -stranded beta-barrel structure characteristic of GFP. The crystal structure of DsRed has also been solved, and shows conservation of the 11-stranded beta-barrel structure of GFP (MMDB Id: 5742).

A number of mutants of the longer wavelength red fluorescent protein DsRed have also been described, and similarly, may be employed in the generation of the fusion proteins of the invention comprising fluorescent (poly)peptides. For example, recently described DsRed mutants with emission spectra shifted further to the red may be employed in the practice of the invention (Baird et al., 2000; Terskikh et al., 2000; Wiehler et al., 2001).

Monomeric versions of Ds Red are e.g. mRFP (e.g. having the amino acid sequence of SEQ ID NO: 5 or the nucleic acid sequence of SEQ ID NO: 6), mRFP1 (Campbell et al., 2002), mCherry, mOrange or mPlum (Shaner et al., 2004) or TagRFP (Merzlyak et al., 2007).
Most recently, GFPs from the anthozoans *Renilla reniformis* and *Renilla kollikeri* were described (Ward et al., U.S. Patent Appn. 20030013849).
An increasingly large number of other fluorescent proteins from a number of ocean life forms have recently been described, and the Protein Data Bank currently lists a number of GFP and GFP mutant crystal structures, as well as the crystal structures of various GFP analogs. Related fluorescent proteins with structures inferred to be similar to GFP from corals, sea pens, sea squirts, and sea anemones have been described, and may be used in the generation of the fusion proteins of the invention comprising fluorescent (poly)peptides (for reviews, see (Zhang et al., 2002; Zimmer, 2002)).
Fluorescent proteins from *Anemonia majano, Zoanthus sp., Discosoma striata, Discosoma sp.* and *Clavularia sp.* have also been reported (Matz et al., 1999). A fluorescent protein cloned from the stony coral species, *Trachyphyllia geoffroyi,* has been reported to emit green, yellow, and red light, and to convert from green light to red light emission upon exposure to UV light (Ando et al., 2002). Recently described fluorescent proteins from sea anemones include green and orange fluorescent proteins cloned from *Anemonia sulcata* (Wiedenmann et al., 2000), a naturally enhanced green fluorescent protein cloned from the tentacles of *Heteractis magnifica* (Tu et al., 2003), a generally non fluorescent purple chromoprotein displaying weak red fluorescence cloned from *Anemonia sulcata* and a mutant thereof displaying far-red shift emission spectra (595nm) (Lukyanov et al., 2000). Additionally, another class of GFP-related proteins having chromophoric and fluorescent properties has been described. One such group of coral-derived proteins, the pocilloporins, exhibit a broad range of spectral and fluorescent characteristics (Dove and Hoegh-Guldberg, 1999, PCT application WO 00146233; (Dove et al., 2001)). Recently, the purification and crystallization of the pocilloporin Rtms5 from the reef-building coral *Montipora efflorescens* has been described (Beddoe et al., 2003). Rtms5 is deep blue in colour, yet is weakly fluorescent. However, it has been reported that Rtms5, as well as other chromoproteins with sequence homology to Rtms5, can be interconverted to a far-red fluorescent protein via single amino acid substitutions (Beddoe et al., 2003; Bulina et al., 2002; Lukyanov et al., 2000).

Various other coral-derived chromoproteins closely related to the pocilloporins are also known (see, for example, (Gurskaya et al., 2001; Lukyanov et al., 2000)). Further examples of fluorescent proteins are GFP form *Renilla reniformis,* mKO from *Fungia concinna,* Azami Green from *Galaxeidae* or cOFP from *Cerianthus.* Any of the fluorescent or chromophoric proteins or fluorescent or chromophoric fragments thereof may be used in accordance with the teaching of the present invention. Fragments of the fluorescent or chromophoric protein are preferably functional fragments.

Accumulation of a (poly)peptide at distinct sites in the nucleus of the cell may be caused by the (poly)peptide interacting with proteinaceous or non-proteinaceous structures already accumulated at distinct sites in the nucleus of the cell or by accumulating at a distinct site providing a suitable environment for the accumulation of the (poly)peptide, preferably by directly or indirectly binding to said sites.

"Co-localization of the fluorescence emission in the nucleus" denotes the localization of two different fluorescence emissions at the same site of the cell nucleus. Co-localization is detected as soon as two proteins interact with each other. Co-localization is detected as the partial or complete spatial overlap of fluorescence emission from two different fluorescent (poly)peptides in the nucleus. Detection can be effected by the experimenter or by specialized software known to the skilled person (e.g. ImageJ co-localization plug-ins, http://rsb.info.nih.gov/ij/).

The present invention relies on the development of a novel fluorescent two-hybrid (F2H) assay for the direct visualization of protein-protein interactions in living eukaryotic cells. The simple optical readout of this F2H assay allows observation of protein-protein interactions in real time and may be employed for high-throughput screens. The method of the invention is based on the immobilization of a fluorescently labeled bait (poly)peptide at a distinct nuclear structure enabling the detection of protein-protein interactions as co-localization of a differently labeled prey (poly)peptide at this defined structure. The F2H assay of the invention was tested on the example of cell lines with a stable integration of a *lac* operator array to immobilize a lac repressor fused to fluorescently labeled (poly)peptides of interest (bait (poly)peptides). Readily usable cell lines have already been described for human, mouse, hamster and *Drosophila* (Dietzel et al., 2004; Janicki et al., 2004; Robinett et al., 1996; Tsukamoto et al., 2000; Tumbar et al., 1999; Vazquez et al., 2001). To be independent of specific transgenic cell lines, this assay could be modified by using various cellular structures like the lamina or centrosomes as anchoring structures to locally immobilize bait (poly)peptides.

Like other genetic two-hybrid methods also the F2H assay of the invention may yield false positive or false negative results, which need to be controlled for. Prey (poly)peptides that bind to the *lac* operator array in the absence of a bait (poly)peptide can be identified by an initial screen. To this end the localization of prey (poly)peptides within the nucleus is determined by fluorescent microscopy in the absence of a respective bait protein. Subsequently, a random accumulation of the fluorescent prey protein at the *lac* operator array can be determined by a clustered fluorescence at this structure. (Poly)peptides identified in this way can only be used as bait (poly)peptides to avoid false positive results. More than 20 protein-protein interactions from different subcellular compartments were analyzed with the F2H assay of the present invention and identical results as previously described with other genetic or biochemical methods were obtained. Proteins found to bind by themselves to the *lac* operator array (such as e.g. SUMO3 discussed in the examples) can only be used as a bait (poly)peptide. The results disclosed in the examples show that the F2H assay of the present invention is a reliable and broadly applicable method to study protein-protein interactions.

In some cases, proteins may accumulate at subnuclear foci and thus complicate the F2H analysis. To bypass this problem, the *lac* operator array could be visualized and identified with a third fluorescent fusion protein like CFP-Lacl.

The present invention is further characterized in that a minimal construct or fusion protein comprising a (poly)peptide that, when expressed in a cell, accumulates at distinct sites in the nucleus of the cell and a (poly)peptide specifically binding to GFP can serve for the rapid and efficient high-throughput screening of protein interactions. Upon co-expression of a second fusion protein comprising GFP and a bait (poly)peptide, the interaction of GFP with the GFP-binding (poly)peptide bound to the (poly)peptide accumulated at distinct sites in the nucleus of the cell is established thus constituting the bait complex. Upon co-expression of a further (third) fusion protein comprising a fluorescent (poly)peptide and a prey (poly)peptide, both fluorescent (poly)peptides co-localize upon interaction of the bait and prey (poly)peptides fused thereto.
Libraries of proteins fused to GFP and of nucleic acids encoding the latter have by now been designed and established, (Newman et al., 2006), which greatly facilitates high-throughput screening for interaction partners of a protein.
In the international patent application WO 2007/068313, the present inventors disclose a (poly)peptide specifically binding to GFP derived from a camel VHH domain. Accordingly, it is preferred that the protein specifically binding to GFP comprises the amino acid sequence as shown in SEQ ID NO: 7 or 9 or encoded by a nucleic acid molecule comprising a sequence as shown in SEQ ID NO: 8 or 10.

In summary, this new F2H assay allows the direct visualization of protein-protein interactions and should be ideally suited to investigate cell cycle or differentiation dependent changes in real-time in living cells. A significant advantage of the F2H assay over other cell-based techniques is its simplicity that does neither require costly instrumentation nor advanced technical expertise. The simple optical read-out of the F2H assay additionally offers the possibility to use this assay in automated high-throughput screens to systematically analyze the protein interactome in living cells.

As compared to the method described in (Miller et al., 2007) which relies on viral structures randomly accumulating in the cytoplasm and forming irregular and unforeseeable structures, the nuclear structures used in the present invention form defined and identifiable spots. In contrast, the method of Miller et al. aims at and results in many poorly defined and large cytoplasmic aggregates which are difficult to distinguish from unspecific aggregates often observed with artificially over-expressed fusion proteins or cytoplasmic vesicles. Using inert structures such as the lac-operator, it is immediately possible to detect unspecific aggregation of a fluorescent (poly)peptide, e.g. by the presence of a varying number of aggregates in the cells. In the method of the present invention, depending on the ploidy of the cell one to two spots are detectable.

In a second aspect, the present invention relates to an in vitro method for identifying a compound modulating the interaction of two (poly)peptides (a) expressing in a eukaryotic cell a first fusion protein comprising (i) a (poly)peptide that, when expressed in a cell, accumulates at distinct sites in the nucleus of the cell and (ii) a (poly)peptide specifically binding to GFP; (b) expressing in the same cell a second fusion protein comprising (i) GFP; and (ii) a bait (poly)peptide; (c) expressing in the same cell a third fusion protein comprising (i) a fluorescent (poly)peptide, the excitation and/or emission wavelength of which differs from that of GFP and (ii) a prey (poly)peptide known or suspected to interact with the bait (poly)peptide; (d) contacting the cell with a test compound; and (e) detecting the fluorescence emission of the fluorescent parts of the second and the third fusion protein in the cell upon excitation; wherein a change in the degree of co-localization of the fluorescence emission of the fluorescent parts of the second and the third fusion protein in the cell nucleus as compared to that observed in the nucleus of a reference cell not contacted with the test compound is indicative that the compound is capable of modulating the interaction of the bait and the prey (poly)peptide.

"Modulating the interaction of two (poly)peptides" denotes the capability of certain compounds to influence the interaction of two (poly)peptides or proteins. The interaction can either be strengthened or weakened up to its complete abrogation. Modulation in the form of a weakening can take place e.g. by competition of a test compound with the interacting protein for the same binding site or by binding to one protein and thus altering its three dimensional structure so that the interaction between the two proteins is no longer possible due to conformational changes in said protein. On the other hand, the latter can also lead to an increase in the binding affinity towards a protein for the same reason.

A test compound can be but is not restricted to a compound belonging to the classes of e.g. nucleic acids, (poly)peptides, peptide aptamers, nucleic acid based aptamers, small molecules or antibodies or fragments thereof. The test compound can be any chemical compound.
Nucleic acids can be DNA, RNA or ribozymes. Nucleic acids can be synthesized chemically or produced in conjunction with a promoter by biological expression *in vitro* or even *in vivo.*

Nucleic acids can be chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL , USA), Glen Research (Sterling, VA, USA), ChemGenes (Ashland, MA, USA), and Cruachem (Glasgow, UK).

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Examples of well-characterized small self-cleaving RNAs are the hammerhead, hairpin, hepatitis delta virus, and in vitro-selected lead-dependent ribozymes. The organization of these small catalysts is contrasted to that of larger ribozymes, such as the group I intron.

Aptamers are oligonucleic acid or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications. More specifically, aptamers can be classified as DNA or RNA aptamers or peptide aptamers. Whereas the former consist of (usually short) strands of oligonucleotides, the latter consist of a short variable peptide domain, attached at both ends to a protein scaffold.

Nucleic acid aptamers are nucleic acid species that have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms. Peptide aptamers are (poly)peptides that are designed to interfere with other protein interactions inside cells. They consist of a variable peptide loop attached at both ends to a protein scaffold. This double structural constraint greatly increases the binding affinity of the peptide aptamer to levels comparable to that of an antibody (nanomolar range). The variable loop length is typically comprised of 10 to 20 amino acids, and the scaffold may be any protein, which has good solubility properties. Currently, the bacterial protein Thioredoxin-A is the most frequently used scaffold protein, the variable loop being inserted within the reducing active site, which is a - Cys-Gly-Pro-Cys- loop in the wild protein, the two cystein lateral chains being able to form a disulfide bridge. Peptide aptamer selection can be made using different systems, but the most frequently used one is currently the yeast two-hybrid system.

An antibody can be, for example, polyclonal or monoclonal. The term "antibody" also comprises derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999.

A small molecule according to the present invention can be organic or inorganic and has a molecular weight of up to 2000 Daltons, preferably not more than 1000 Daltons, most preferably not more than 800 Daltons.

In a preferred embodiment of this aspect of the present invention, the test compound is capable of weakening the interaction of two (poly)peptides, wherein a decrease of co-localization of the fluorescent parts of both fusion proteins in the cell nucleus as compared to that observed in the nucleus of a reference cell not contacted with the test compound is indicative of the compound being capable of weakening the interaction of the bait and the prey (poly)peptide.

"Capable of weakening the interaction of two (poly)peptides" in context with the present invention means firstly the influence of a compound on the binding affinity of a (poly)peptide towards another (poly)peptide leading to a weakening or complete disruption/abrogation of the interaction or binding affinity. As described above, this may take place by conformational changes induced in one (poly)peptide upon binding of the compound. Secondly, the compound may directly compete with the interacting (poly)peptide for the same or a different binding site adjacent to said binding site on the other (poly)peptide. A weakening or complete abrogation of interaction is detectable as lessening or complete absence of co-localization of both fluorescent signals.

A reference cell in the context of the present invention denotes a cell that has not been contacted with the test compound. Accordingly, the influence of the compound on the interaction behaviour of the bait and prey (poly)peptide can be compared directly.

In another preferred embodiment of this aspect of the invention, the test compound is capable of inducing or enhancing the interaction of two (poly)peptides, wherein an increase in co-localization of the fluorescent parts of both fusion proteins in the cell nucleus as compared to that observed in the nucleus of a reference cell not contacted with the test compound is indicative of the compound being capable of inducing or enhancing the interaction of the bait and the prey (poly)peptide.

"Capable of inducing the interaction of two (poly)peptides" denotes the influence of a compound on the binding affinity of a (poly)peptide towards another (poly)peptide leading to an increased binding affinity . For example, if no or only minor co-localization of the fluorescent signals of the bait and the prey (poly)peptide are detected in the reference cell, an increase in co-localization indicates a strengthened interaction.

In a different aspect, the present invention relates to an in vitro method of determining the relative strength of the interaction of two proteins with a third protein comprising: (a) expressing in a eukaryotic cell a first fusion protein comprising (i) a (poly)peptide that, when expressed in a cell, accumulates at distinct sites in the nucleus of the cell; and (ii) a (poly)peptide specifically binding to GFP; (b) expressing in the same cell a second fusion protein comprising (i) GFP; and (ii) a bait (poly)peptide; (c) expressing in the same cell a third fusion protein comprising (i) a fluorescent (poly)peptide, the excitation and/or emission wavelength of which differs from that of GFP; and (ii) a first prey (poly)peptide (d) expressing in the same cell a fourth fusion protein comprising (i) a fluorescent (poly)peptide, the excitation and/or emission wavelength of which differs from that of GFP and from that of the fluorescent (poly)peptide of said third fusion protein; and (ii) a second prey (poly)peptide; and (e) detecting the fluorescence emission of the fluorescent parts of the second and the third fusion protein in the cell upon excitation, wherein an extent of co-localization of the fluorescence emission of the second and the third fusion protein different as compared to that of the second and the fourth fusion protein in the cell nucleus is indicative of a different binding strength of the first and the second prey (poly)peptide to the bait (poly)peptide.

This and the following two aspects of the invention rely on two basic constructs, a prey and a bait construct, and can be applied in cases where libraries of nucleic acids encoding proteins comprising GFP are not available.

In a further aspect, the present invention relates to an in vitro method for detecting protein-protein interactions comprising: (a) expressing in a eukaryotic cell a first fusion protein comprising (i) a fluorescent (poly)peptide; (ii) a (poly)peptide that, when expressed in a cell, accumulates at distinct sites in the nucleus of the cell; and (iii) a bait (poly)peptide; (b) expressing in the same cell a second fusion protein comprising (i) a fluorescent (poly)peptide, the excitation and/or emission wavelength of which differs from that of the fluorescent (poly)peptide comprised in said first fusion protein; and (ii) a prey (poly)peptide and (c) detecting the fluorescence emission of the fluorescent parts of the first and the second fusion protein in the cell upon excitation, wherein a co-localization of the fluorescence emission of both fusion proteins in the cell nucleus is indicative of an interaction of the bait and the prey (poly)peptide.

In a different aspect, the present invention relates to an in vitro method for identifying a compound modulating the interaction of two (poly)peptides (a) expressing in a eukaryotic cell a first fusion protein comprising (i) a fluorescent (poly)peptide; (ii) a (poly)peptide that, when expressed in a cell, accumulates at distinct sites in the nucleus of the cell; and (iii) a bait (poly)peptide; (b) expressing in the same cell a second fusion protein comprising (i) a fluorescent (poly)peptide, the excitation and/or emission wavelength of which differs from that of the fluorescent (poly)peptide comprised in said first fusion protein; and (ii) a prey (poly)peptide known or suspected to interact with the bait (poly)peptide; (c) contacting the cell with a test compound; and (d) detecting the fluorescence emission of the fluorescent parts of the first and the second fusion protein in the cell upon excitation; wherein a change in the degree of co-localization of the fluorescence emission of the fluorescent parts of both fusion proteins in the cell nucleus as compared to that observed in the nucleus of a reference cell not contacted with the test compound is indicative that the compound is capable of modulating the interaction of the bait and the prey (poly)peptide.

In a further aspect, the present invention relates to a method of determining the relative strength of the interaction of two proteins (interchangeably used with the term "(poly)peptides") with a third protein (or (poly)peptide) comprising (a) expressing in a eukaryotic cell a first fusion protein comprising (i) a fluorescent (poly)peptide; (ii) a (poly)peptide that, when expressed in a cell, accumulates at distinct sites in the nucleus of the cell; and (iii) a bait (poly)peptide; (b) expressing in the same cell a second fusion protein comprising (i) a fluorescent (poly)peptide, the excitation and/or emission wavelength of which differs from that of the fluorescent (poly)peptide comprised in said first fusion protein; and (ii) a first prey (poly)peptide; (c) expressing in the same cell a third fusion protein comprising (i) a fluorescent (poly)peptide, the excitation and/or emission wavelength of which differs from that of the fluorescent (poly)peptide comprised in said first and second fusion protein; and (ii) a second prey (poly)peptide; and (d) detecting the fluorescence emission of the fluorescent parts of the first, the second and the third fusion protein in the cell upon excitation, wherein an extent of co-localization of the fluorescence emission of the first and the second fusion protein different as compared to that of the first and the third fusion protein in the cell nucleus is indicative of a different binding strength of the first and the second prey protein to the bait (poly)peptide.

In a preferred embodiment of the aspects of the present invention relating to methods for detecting protein-protein interactions, the detection is employed for investigating the dependency of protein-protein interactions on cellular processes, wherein the method further comprises (d1) monitoring the fluorescence emission of the fluorescent parts of the first and the second fusion protein in the cell in the course of one or more processes in the cell; wherein a change in the degree of co-localization of the fluorescence emission of the fluorescent parts of both fusion proteins in the cell is indicative of a dependency of the interaction on the one or more cellular processes.

"Dependency on cellular processes" denotes the possibility that the bait or prey (poly)peptide or both alter their interaction behaviour due to modifications in the course of cellular processes such as the cell cycle. Proteins playing a role in the cell cycle undergo various modifications such as phosphorylation and dephosphorylation which might alter their binding affinity towards other proteins. Depending on the time point or period during a cellular process when this modification is effected, a (poly)peptide might loose or gain binding affinity to one or more other (poly)peptide.

In a more preferred embodiment, the cellular process is the cell cycle, secretion, translocation or signal transduction.

In another preferred embodiment, the detection is employed for determining the strength of a protein-protein interaction, wherein the method further comprises (d2) in case that a co-localization of the fluorescence emission of the fluorescent parts of both fusion proteins is detected, selective extinction of the fluorescence of said second fusion protein and monitoring the restoration of co-localization of the fluorescence emission of the fluorescent parts of both fusion proteins over time, wherein the time needed to establish co-localization is indicative of the strength of the protein-protein interaction.

This embodiment of the present invention in part utilizes the well-established FRAP (fluorescence recovery after photobleaching) method. This method denotes an optical technique capable of quantifying the diffusion and mobility of fluorescently labelled probes. This technique provides a great utility in biological studies of protein binding and is commonly used in conjunction with fluorescent proteins (FP), where the studied protein is fused to an FP. When excited by a specific wavelength of light (typically with a laser beam), the protein will fluoresce. When the protein that is being studied is produced with the FP, then the fluorescence can be tracked. After photodestruction of the FP (typically with a strong/intense laser pulse), the kinetic of fluorescence recovery in the bleached area provides information about strength of protein interactions, organelle continuity and protein trafficking that prevents or slows down the exchange of bleached and unbleached FPs. This observation has most recently been exploited to investigate protein binding.

In another preferred embodiment of the present invention, components (i), (ii) and/or (iii) of said first fusion protein and/or components (i) and (ii) of said second fusion protein and/or components (i) and (ii) of said third fusion protein are connected via a linker.

The term "linker" refers to the connection between the components of the fusion proteins of the invention. A linker can be a peptide bond or a stretch of amino acids comprising at least one amino acid residue which may be arranged between the components of the fusion proteins in any order. Such a linker may in some cases be useful, for example, to improve separate folding of the individual domains or to modulate the stability of the fusion protein. Moreover, such linker residues may contain signals for transport, protease recognition sequences or signals for secondary modification. The amino acid residues forming the linker may be structured or unstructured. Preferably, the linker may be as short as 1 amino acid residue or up to 2, 3, 4, 5, 10, 20 or 50 residues. In particular cases, the linker may even involve up to 100 or 150 residues.

In another preferred embodiment of the present invention, the (poly)peptide that, when expressed in a cell, accumulates at distinct sites in the nucleus of the cell directly interacts with proteinaceous or non-proteinaceous structures accumulated at distinct sites in the nucleus of the cell.

In a different preferred embodiment of the present invention, the (poly)peptide that, when expressed in a cell, accumulates at distinct sites in the nucleus of the cell indirectly interacts with proteinaceous or non-proteinaceous structures accumulated at distinct sites in the nucleus of the cell.
Indirect interaction may again occur via proteinaceous or non-proteinaceous molecules such as (poly)peptides or nucleic acids.

In a different preferred embodiment any of the prey (poly)peptide comprises a nuclear localization signal.

A nuclear localization signal targets an expression product to the cell nucleus. An example of an NLS is the peptide sequence PKKKRKV (nuclear-localization signal (NLS) of the SV40 large T-antigen, (Kalderon et al., 1984) which is capable of directing heterologous proteins into the nucleus. Further NLS are for example KR[PAATKKAGQA]KKKK, the NLS of nucleoplasmin as a prototype of an ubiquitous bipartite signal or KIPIK the NLS of the yeast transcription repressor Matα2. Many other sequences of nuclear localization signals are known to the skilled person and described in the literature.
An NLS needs to be present if the prey (poly)peptide fused to the fluorescent (poly)peptide is per se not able to translocate to the nucleus. Generally, (poly)peptides of a size of up to 60 kDa may translocate to the nucleus. Furthermore, the amino acid composition of the (poly)peptide plays a role, i.e. a number of consecutive basic amino acids in a (poly)peptide may promote translocation of the (poly)peptide into the nucleus. In order to ensure that the prey (poly)peptide is translocated into and accumulated in the nucleus, it is preferred that an NLS is present. The same holds true for (poly)peptides accumulating at distinct sites of the nucleus but which only translocate to the nucleus if an NLS is present. An example for such a (poly)peptide is the lac repressor which by itself does not comprise an NLS.
In a different preferred embodiment, expression in the eukaryotic cell is effected by transfecting the nucleic acid molecules encoding said first and second fusion protein in one or more vectors.

Transfection is the introduction of nucleic acid molecules into eukaryotic cells. Commonly used methods comprise but are not restricted to transfection using calcium chloride, JetPEI™(PolyPlus) or lipofectin™ (Invitrogen).

The vector comprising the nucleic acid molecule encoding said first and/or second and/or third and/or fourth fusion protein is a eukaryotic expression vector, preferably a mammalian expression vector. Incorporation of the nucleic acid molecule into a vector offers the possibility of introducing the nucleic acid molecule efficiently into the cells and preferably the DNA of a host cell. The host cell may be a single cell such as a cell from a cell line. Such a measure renders it possible to express, if expression vectors are chosen, the respective nucleic acid molecule in the host cell. Thus, incorporation of the nucleic acid molecule into an expression vector opens up the way to a permanently elevated level of the encoded (poly)peptide or protein in any cell or a subset of selected cells of the host cell.

In another preferred embodiment, the (poly)peptide accumulated at distinct sites of the nucleus of the cell has been introduced into the cell. In a different preferred embodiment in case said (poly)peptide interacts with proteinaceous or non-proteinaceous structures accumulated at distinct sites in the nucleus, said proteinaceous or non-proteinaceous structures can be introduced into the cell. Similarly to the vector(s) comprising the nucleic acid molecules encoding the fusion proteins of the present invention, also the nucleic acid molecules encoding the proteinaceous or non-proteinaceous structures can be introduced in a vector. The nucleic acid molecule is preferably stably integrated into the chromosomes of the cell leading to the generation of a stable cell line.
In a different preferred embodiment, the distinct sites of the cell form inert structures.

In a more preferred embodiment, the inert structure is the nuclear lamina or nuclear speckles.

In a different preferred embodiment, the (poly)peptide that, when expressed in a cell, accumulates at distinct sites in the nucleus of the cell interacts with PML bodies (also termed PML nuclear bodies or PML NBs).
PML bodies have been associated with many nuclear functions including transcription, DNA repair, viral defence, stress, cell cycle regulation, proteolysis and apoptosis. The average mammalian cell contains 10-30 PML nuclear bodies. PML bodies are defined by the presence of the PML protein, first identified by its fusion to the retinoic acid receptor alpha in chromosomal translocation t(15,17) (Borden, 2002; Maul et al., 2000; Moller et al., 2003) associated with acute promyelocytic leukaemia (APL). PML protein is essential for the formation of PML NBs, and when it is absent, or its RING (comprising C₃HC₄ zinc finger the as a structural motif) fingers mutated, PML bodies are disrupted.

In a different preferred embodiment, the (poly)peptide accumulating at distinct sites of the nucleus or the proteinaceous or non-proteinaceous structures accumulated at distinct sites of the cell is heterologous.
"Heterologous" as used in the present invention denotes the origin of a proteinaceous or non-proteinaceous structure which is different from that of the cell in which it is expressed, i.e. a different species.

In another preferred embodiment, the (poly)peptide that, when expressed in a cell, accumulates at distinct sites in the nucleus of the cell interacts with DNA.

In a more preferred embodiment, the DNA is the lac-operator.

In an even more preferred embodiment, the *lac* operator is present in the nucleus in multiple copies. The number of copies depends on the individual experiment, i.e. on the kind of host cell used or the bait (poly)peptide and/or the prey (poly)peptide.

Commonly applied copy number reach from at least 150 copies of a plasmid comprising 256 copies of the *lac* operator (i.e. 38400 copies of the *lac* operator) to about 2000 copies of said plasmid (i.e. 512000 copies of the *lac* operator). In case a higher sensitivity is needed, the copy number can be even higher and reach up to 3000 copies. Alternatively, any kind of plasmid comprising a different copy number can be utilized to obtain the desired copy numbers of the *lac* operator. The *lac* operator can be arranged in tandem with or without nucleic acid stretches separating each element. The elements can be arranged head to tail or head to head. Cell lines having multiple copies of the *lac* operator stably integrated in the nucleus are known in the art (Janicki et al., 2004; Tsukamoto et al., 2000).

In another preferred embodiment, said first fusion protein comprises LacI.

The *lac* repressor LacI is a tetramer of identical subunits. Each subunit contains a helix-turn-helix (HTH) motif capable of binding to DNA. The operator site where the repressor binds is a DNA sequence with inverted repeat symmetry. The two DNA half-sites of the operator bind to two of the subunits of the tetrameric repressor. Both the *lac* operator and LacI form part of the bacterial lac-operon, a regulatory unit for lactose metabolism of bacteria. If lactose is missing from the growth medium, the repressor binds very tightly to the lac operator located downstream of the promoter. The present invention makes use of the interaction between LacI and the lac operator in order to form a non-proteinaceous anchor structure in the nucleus with which the method of the invention can be conveniently carried out. Generally, the interaction of LacI with the lac operator is detected at one distinct site in the nucleus.

An important advantage of this embodiment of the present invention as compared to previously known methods, in particular that of (Miller et al., 2007) is that the copy number of the lac operator forming a lac operator array can be varied in order to adapt the system to the expression level of both the bait and the prey (poly)peptides. For example, if the expression of one or both (poly)peptides is found too high in the cell, thus e.g. interfering with cellular processes or disturbing the fluorescent signal, another cell having a lower copy number of the lac operator can be taken, thus enabling for a titration of the detection of the interaction.

In a more preferred embodiment, said second fusion protein comprises GFP.

In another preferred embodiment, the detection is carried out using a fluorescence microscope.

A fluorescence microscope is a light microscope used to study properties of organic or inorganic substances using the phenomena of fluorescence and phosphorescence instead of, or in addition to, reflection and absorption. The specimen is illuminated with light of a specific wavelength (or wavelengths) which is absorbed by the fluorophores, causing them to emit longer wavelengths of light (of a different color than the absorbed light). The illumination light is separated from the much weaker emitted fluorescence through the use of an emission filter. Typical components of a fluorescence microscope are the light source (Xenon or Mercury arc-discharge lamp), the excitation filter, the dichroic mirror (or dichromatic beamsplitter), and the emission filter. The filters and the dichroic mirror are chosen to match the spectral excitation and emission characteristics of the fluorophore used to label the specimen. Most fluorescence microscopes in use are epi-fluorescence microscopes (i.e.: excitation and observation of the fluorescence are from above (epi) the specimen). These microscopes have become an important part in the field of biology, opening the doors for more advanced microscope designs, such as the confocal laser scanning microscope (CLSM) and the total internal reflection fluorescence microscope (TIRF). These technologies are well known to the skilled person.

In another preferred embodiment, the eukaryotic cell is a living cell.

In another preferred embodiment, the method of the invention further comprises localizing the bait and/or prey (poly)peptides in the cell.
This embodiment serves to confirm that the co-localization effectively takes place in the nucleus and to rule out unspecific interaction of the bait and prey (poly)peptides. Localization is either detected by the experimenter or by specialized software able to distinguish different cellular compartments and well-known to the skilled person (e.g. ImageJ (Version 1.38, http://rsb.info.nih.gov/ij/).

Also described herein is a nucleic acid molecule encoding a fusion protein comprising (i) a fluorescent (poly)peptide; (ii) a (poly)peptide that, when expressed in a cell, accumulates at distinct sites in the nucleus of the cell: and (iii) a bait (poly)peptide.

Also described herein is a nucleic acid molecule encoding a fusion protein comprising (i) a (poly)peptide that binds to the lac-operator; and (ii) a (poly)peptide specifically binding to GFP, a vector comprising said nucleic acid and a protein encoded by said nucleic acid.

A "nucleic acid molecule" includes DNA, such as cDNA or genomic DNA, and RNA, both sense and anti-sense strands. Further included are nucleic acid mimicking molecules known in the art such as synthetic or semi-synthetic derivatives of DNA or RNA and mixed polymers. Such nucleic acid mimicking molecules or nucleic acid derivatives include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see (Braasch and Corey, 2001)). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art.

Further described herein is that the fusion protein comprises LacI.

Also described herein is a protein encoded by the nucleic acid molecule of the invention. The features of a protein or (poly)peptide are defined elsewhere in this application.

It is further described that the protein comprises LacI.

In an alternative aspect, the present invention relates to a vector comprising the nucleic acid molecules as characterised in the appended claims. The properties of a vector have been described elsewhere in this application. In addition to the features already described, the vector of the present invention can be a prokaryotic vector. Prokaryotic vectors and their properties are well known in the art. Preferably, the vector is a plasmid, cosmid, virus, bacteriophage or another vector conventionally used e.g. in genetic engineering.

The nucleic acid molecule may be inserted into several commercially available vectors. Non-limiting examples include vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen), pCINeo (Promega), Okayama-Berg cDNA expression vector pcDV1 (Pharmacia), pRc/CMV, pcDNA1, pSPORT1 (GIBCO BRL), pGEMHE (Promega) or pSVL and pMSG (Pharmacia, Uppsala, Sweden).

For vector modification techniques, see Sambrook and Russel (2001), loc. cit. Generally, vectors can contain one or more origin of replication (ori) and inheritance systems for cloning or expression, one or more markers for selection in the host, e. g., antibiotic resistance, and one or more expression cassettes.

The nucleic acid molecules inserted in the vector can e.g. be synthesized by standard methods, or isolated from natural sources. Ligation of the coding sequences to transcriptional regulatory elements and/or to other amino acid encoding sequences can be carried out using established methods.
In accordance with the present invention, the vector further comprises a nucleic acid molecule encoding a fusion protein comprising (i) a fluorescent (poly)peptide, the excitation and/or emission wavelength of which differs from that of the fluorescent (poly)peptide comprised in the protein encoded by the nucleic acid molecule described above and (ii) a prey (poly)peptide.

In a different aspect, the present invention relates to a eukaryotic cell transfected with the vector of the invention.

It is preferred that the eukaryotic cell is a mammalian cell.

In another aspect of the invention, a eukaryotic cell as characterised in the appended claims is transfected with a vector comprising the nucleic acid molecule described above and with a vector comprising a nucleic acid molecule encoding a fusion protein comprising (i) a fluorescent (poly)peptide, the excitation and/or emission wavelength of which differs from that of the fluorescent (poly)peptide comprised in the protein described above and (ii) a prey (poly)peptide.

Further described herein is a eukaryotic cell having multiple copies of the lac-operator stably integrated in its DNA and stably expressing the protein of the invention.

In another aspect, the invention relates to a eukaryotic cell having multiple copies of the lac-operator stably integrated in its DNA and stably expressing a protein comprising (i) a (poly)peptide that binds to the lac operator; and (ii) a (poly)peptide specifically binding to GFP.

In a preferred embodiment, said (poly)peptide specifically binding to GFP comprises a (poly)peptide having the amino acid sequence of SEQ ID NOs: 7 or 9 or encoded by the nucleic acid sequence of SEQ ID NOs: 8 or 10.

Also described herein is a nucleic acid molecule encoding a fusion protein comprising (i) a (poly)peptide that, when expressed in a cell, accumulates at distinct sites in the nucleus of the cell; and (ii) a (poly)peptide specifically binding to GFP; as well as the protein encoded by said nucleic acid molecule, a vector comprising said nucleic acid molecule and a eukaryotic cell transfected with the vector comprising said nucleic acid molecule. The preferred embodiments and definitions as described for the aspects of the present invention relating to an F2H system comprising a bait and at least one prey construct are equally applicable to the above aspects utilizing the (poly)peptide specific for GFP. All embodiments relating to the first, second or third fusion protein of the first three aspects of the invention equally apply to the four fusion proteins of the aspects utilizing the (poly)peptide specific for GFP.

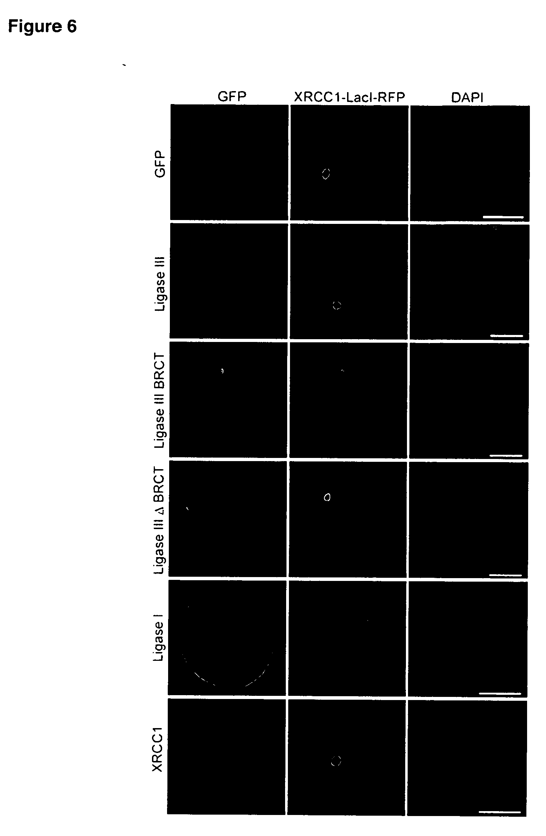

The figures show:
**Figure 1**
   Schematic outline of the fluorescent two-hybrid (F2H) assay. (a) Outline of pF2H-prey and pF2H-bait expression vectors coding for fluorescently labeled prey- and bait- proteins used for the F2H assay (b) The LacI domain of the bait-protein mediates binding to the chromosomally integrated *lac* operator array, which is visible as a fluorescent spot in nuclei of transfected cells. If the differentially labeled prey interacts with the bait it becomes enriched at the same spot resulting in co-localization of fluorescent signals at the *lac* operator (visible as yellow spot in the overlay image). (c) If the prey does not interact with the bait protein it remains dispersed in the nucleus and the *lac* operator array is only visualized by the bait protein (red spot). FP1 and FP2 refer to two distinguishable fluorescent proteins, e.g. GFP or YFP and mCherry or mRFP.
**Figure 2**
   Specific interaction of DNA Ligase III with XRCC1 revealed by F2H (a) Transgenic BHK cells containing a chromosomal *lac* operator array were co-transfected with XRCC1-LacI-RFP and GFP-tagged DNA Ligase III or DNA Ligase I constructs. The lac repressor part of the XRCC1-LacI-RFP fusion protein mediates binding to the *lac* operator array (visible by fluorescence microscopy as red spot). DNA Ligase III is recruited to the *lac* operator array through interaction with XRCC1. Note that the highly homologous DNA Ligase I does not accumulate at the *lac* operator array indicating that it does not interact with XRCC1. Scale bars 5 µm. (b) Comparison of F2H results and co-immunoprecipitation (Co-IP) experiments. Co-IPs were performed with HEK 293T cells co-expressing RFP-XRCC1 and GFP-Ligase III or GFP-Ligase I, respectively. For interaction mapping the GFP-tagged BRCT domain of DNA Ligase III and a deletion construct lacking the BRCT domain were used. Immunoprecipitations were performed with a GFP-nanotrap (Rothbauer et al., 2007) (as shown before (Mortusewicz et al., 2006)). Precipitated fusion proteins were then detected with specific antibodies against RFP and GFP on western blots. RFP-XRCC1 was co-precipitated with GFP-Ligase III but not with GFP-Ligase I. RFP-XRCC1 was also co-precipitated with GFP-Ligase III BRCT but not with GFP-N-Ligase III ΔBRCT. For comparison of F2H results the input (left) and bound (right) bands from Co-IPs were aligned with corresponding signals from the F2H assay. The LacI spot of the XRCC1-LacI-RFP bait construct shown in red and the bound fraction was aligned with the respective signal of the GFP-tagged prey constructs. Whole cell images of the respective F2H experiments are shown in (a) and Figure 6.
**Figure 3**
   F2H analysis of cell cycle independent interaction of Dnmt1 with PCNA. (a) Schematic outline of full-length mouse Dnmt1 and fusion proteins. PBD, PCNA binding domain; NLS, nuclear localization sequence; TS, targeting sequence; ZnF, Zn²⁺-binding region; BAH 1 and 2, two Bromo Adjacent Homology domains. (b) Outline of binding possibilities of fusion proteins at the *lac* operator (*lac* op) array and at the replication fork. (c) Transgenic BHK cells containing a chromosomal *lac* operator array were co-transfected with PBD-LacI-YFP and RFP-PCNA constructs. RFP-PCNA shows the characteristic cell cycle dependent distribution (dispersed in non S phase cells (top row) and focal patterns in S phase (bottom row)). The lac repressor part of the PBD-LacI-YFP fusion protein mediates binding to the *lac* operator array (visible as green spot and highlighted by arrowheads) and the PBD mediates binding to PCNA at replication sites (focal pattern in S phase). Notice, RFP-PCNA is localized at the *lac* operator array in S and non S phase cells indicating an interaction of the PBD of Dnmt1 with PCNA throughout the cell cycle and independent of the replication machinery. (d) BHK cells were transfected with expression vectors for ΔPBD-LacI-YFP and RFP-PCNA. As above, RFP-PCNA shows a disperse distribution in non S phase (top row) and redistributes to replication sites in S phase (bottom row). The ΔPBD-LacI-YFP fusion protein binds to the *lac* operator array (green spot marked by arrowhead) but does not bind to replication sites in S phase since it lacks the PBD. Importantly, in these cells RFP-PCNA (prey) is not localized at the *lac* operator array (marked by arrowheads) indicating that binding depends on the presence of the PBD, which is absent in ΔPBD-LacI-YFP (bait). Scale bars 5 µm
**Figure 4**
   Analysis of Huntington's disease related interactions by F2H. Reported interactions between (a) SUMO3 and HZFH and (b) HZFH and Vimentin revealed by F2H. (c) F2H analysis shows no interaction between SUMO3 and Vimentin as previously described (Goehler et al., 2004). In (b) the nucleus is outlined by a dashed line and in (c) the *lac* operator array is indicated (arrowheads). Scale bars 5 µm.
**Figure 5**
   Analysis of mitochondrial protein-protein interactions and the effect of a mutation associated with the Mohr-Tranebjaerg Syndrome. (a) Schematic overview of the hexameric DDP1-TIMM13 complex in the intermembrane space (IMS) of mitochondria. (b + c) BHK cells expressing the bait-protein mCherry-LacI-TIMM13 together either with GFP-DDP1 (b) or the loss-of-function mutant GFP-DDP1^{C66W} (c). While the functional wt fusion GFP-DDP1 shows interaction with TIMM13 revealed by co-localization of fluorescent signals at the *lac* operator array (b), the GFP-DDP1^{C66W} mutant is dispersedly distributed throughout the nucleus indicating no interaction (c). Scale bars 5 µm.
**Figure 6**
   BRCT mediated interaction of DNA-Ligase III with XRCC1 revealed by the F2H assay. Transgenic BHK cells containing a *lac* operator array were co-transfected with XRCC1-LacI-RFP and various GFP-tagged DNA-Ligase III constructs. The lac repressor part of the XRCC1-LacI-RFP fusion protein mediates binding to the *lac* operator array (visible as red spot). The BRCT domain is necessary and sufficient for targeting of DNA Ligase III to the *lac* operator array through interaction with XRCC1. Note that the highly homologous DNA Ligase I does not accumulate at the *lac* operator array indicating that it does not interact with XRCC1. Scale bars 5 µm.
**Figure 7**
   The F2H assay reveals the interaction of XRCC1 with PCNA, PARP-1 and PARP-2. BHK cells containing a *lac* operator array were transfected with expression vectors for XRCC1-LacI-RFP and either GFP-PARP-1, GFP-PARP-2 or GFP-PCNA. The lac repressor part of the XRCC1-LacI-RFP fusion protein mediates binding to the *lac* operator array (visible as red spot). GFP-PARP-1, GFP-PARP-2 and GFP-PCNA are targeted to the *lac* operator array indicating an interaction with XRCC1.Scale bar 5 µm.
**Figure 8**
   The F2H assay reveals the PBD-mediated interaction of DNA-Ligase I with PCNA. Transgenic U2OS cells containing a *lac* operator array were co-transfected with NLS-PCNA-Lacl-RFP and various GFP-tagged DNA-Ligase I constructs. The lac repressor part of the NLS-PCNA-Lacl-RFP fusion protein mediates binding to the *lac* operator array (visible as red spot). The PBD is necessary and sufficient for targeting of DNA Ligase I to the *lac* operator array through interaction with PCNA. Scale bar 5 µm.
**Figure 9**
   Interaction of various replication and repair proteins with PCNA revealed by the F2H assay. Transgenic BHK cells containing a *lac* operator array expressing NLS-PCNA-Lacl-RFP and various GFP-tagged replication and repair proteins. All proteins tested interact with PCNA. Scale bar 5 µm.

The examples illustrate the invention.

### Example 1: Materials and Methods

### Expression constructs

The LacI encoding sequence was PCR amplified from the p3'SS EGFP-Lacl expression vector (Robinett et al., 1996) using the following primers: forward primer 5'-TCT AGA *AAG CTT* TCC ATG GTG AAA CCA GTA-3' and reverse primer 5'-CCA TGC CCG GGA CAG GCT GCT TCG GGA AAC-3' (restriction sites in italic). This PCR fragment was digested with Hindlll and Xmal and cloned into the same sites of two Dnmt1-YFP expression vectors (MTNY.2 and PBHD-YFP) (Easwaran et al., 2004) generating PBD-Lacl-YFP and ΔPBD-LacI-YFP. The NLS-PCNA-Lacl-RFP and XRCC1-LacI-RFP constructs were generated by PCR amplification of the PCNA and XRCC1 cDNA using the following primers (restriction sites in italic):

| | |
|---|---|
| PCNA forward | 5'- CCCCCTCGAGATGTTCGAGGCGCGC -3' |
| PCNA reverse | 5'- GGGGAAGCTTGGAGATCCTTCTTCATCCTC- 3' |
| XRCC1 forward | 5'- CCCCAGATCTATGCCGGAGATCCGC -3' |
| XRCC1 reverse | 5'- GGGGGAATTCGGGGCTTGCGGCACCAC -3' |

Subsequently the PCR fragments were cloned into a LacI-RFP expression vector using the XhoI/HindIII sites for the NLS-PCNA-LacI-RFP and the BgIII/EcoRI sites for the XRCC1-LacI-RFP expression vector.

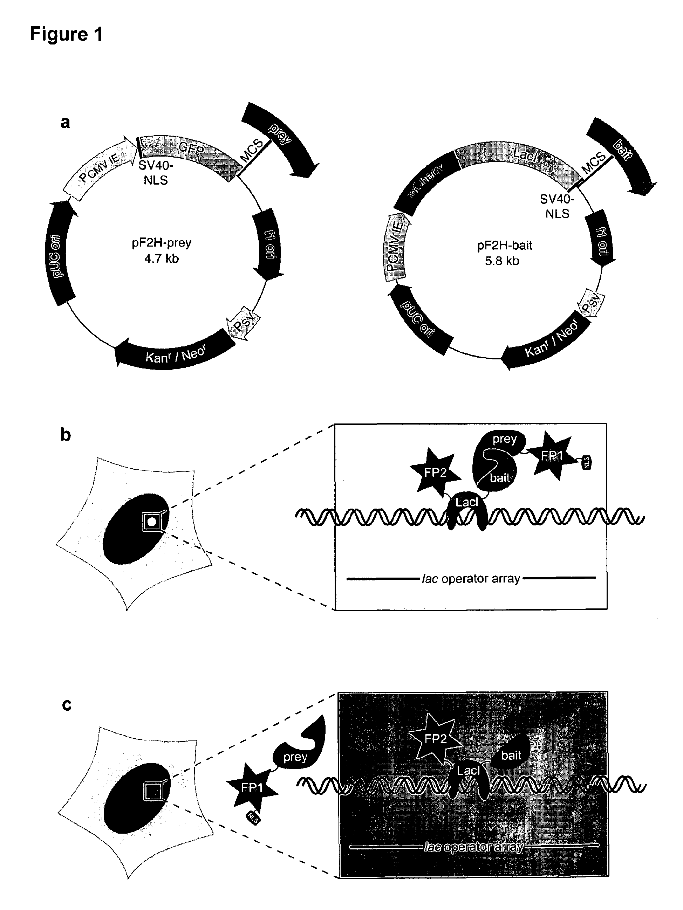

All other F2H constructs were generated by PCR amplification of coding cDNAs and subsequent ligation into the AsiSI and Notl sites of the bait and prey expression vectors described in Figure 1 a. The following primers were used with the restriction site indicated in italics:

| | |
|---|---|
| DDP1 forward | 5'-CCCCGCGATCGCGATTCCTCCTCCTCTTCCTC-3' |
| DDP1 reverse | 5'-CCCCGCGGCCGCTCAGTCAGAAAGGCTTTCTG-3' |
| TIMM13 forward | 5'-CCCCGCGATCGCGAGGGCGGCTTCGGCTCC-3' |
| TIMM13 reverse | 5'-CCCCGCGATCGCGAGGGCGGCTTCGGCTCC-3' |
| HZFH forward | 5'-GGGGGCGATCGCCACGCCCGCTTCC-3' |
| HZFH reverse | 5'-CCCCGCGGCCGCTTAGTCGTCTATACAGATCACCTCC-3' |
| SUMO3 forward | 5'-CCCCGCGATCGCGCCGACGAAAAGCCCAAG-3' |
| SUMO3 reverse | 5'-CCCCGCGGCCGCTCAGTAGACACCTCCCG-3' |
| Vim forward | 5'-GGGGTGTACAGCGA TCGCATGTCGACCCACGCGT-3' |
| Vim reverse | 5'-CCCCGAATTCGCGGCCGCTTATTCAAGGTCATCGTGATGCT-3' |

Mammalian expression constructs encoding translational fusions of human DNMT1, DNA-Ligase I, DNA-Ligase III, p21, FEN I, Polymerase δ p66 subunit, PARP-1, PARP-2 and PCNA were previously described (Cazzalini et al., 2003; Maeda et al., 2006; Meder et al., 2005; Mortusewicz et al., 2005; Schermelleh et al., 2005; Sporbert et al., 2005). Deletion constructs and isolated domains of DNA-Ligase I and III were described in Mortusewicz et al (Mortusewicz et al., 2006). Immunoprecipitations were performed with a GFP-nanotrap (Rothbauer et al., 2007) as described before(Mortusewicz et al., 2006). All fusions constructs were tested for correct expression and localization.

### Cell culture and transfection

Transgenic BHK cells (clone #2) and U2OS cells (clone 2-6-3) containing *lac* operator repeats were cultured under selective conditions in DMEM supplemented with 10% fetal calf serum and 150 µg/ml hygromycin B (PAA Laboratories) as described (Janicki et al., 2004; Tsukamoto et al., 2000). For microscopy cells were grown to 50-70% confluence either on 18x18 glass coverslips or in µ-slides (ibidi, Munich, Germany) and then co-transfected with the indicated expression constructs using Polyplus transfection reagent jetPEI™ (BIOMOL GmbH, Hamburg, Germany) according to the manufacturer's instructions. After 6-10 hours the transfection medium was changed to fresh culture medium and cells were then incubated for another 12-24 hours before live cell microscopy or fixation with 3.7 % formaldehyde in PBS for 10 min at room temperature. Fixed cells were permeabilized with 0.2 % Triton X-100 in PBS for 3 min, counterstained with DAPI and mounted in Vectashield (Vector Laboratories, CA, USA).

### Microscopy

Live or fixed cells expressing fluorescent proteins were analyzed using a Leica TCS SP2 AOBS confocal microscope equipped with a 63x/1.4 NA Plan-Apochromat oil immersion objective. Fluorophores were excited with a 405 nm Diode laser, a 488 nm and a 514 nm argon laser and a 561 nm Diode-Pumped Solid-State (DPSS) laser. Confocal image stacks of living or fixed cells were typically recorded with a frame size of 512x512 pixels, a pixel size of 50-100 nm, a z-step size of 250 nm and the pinhole opened to 1 Airy unit. A maximum intensity projection of several mid z-sections was generated with ImageJ (Version 1.38, http://rsb.info.nih.gov/ij/).

### Example 2: Method to detect the interaction of proteins

To visualize protein-protein interactions in living cells in real time we developed a fluorescence two-hybrid (F2H) assay. The rationale for the F2H assay is based on the fact that proteins are freely roaming the cell unless interactions with other cellular components immobilize them at specific structures (Phair and Misteli, 2000). We used a previously described BHK and an U2OS cell line which both harbor a stable integration of about 200-1000 copies of a plasmid carrying 256 copies of the *lac* operator sequence (Janicki et al., 2004; Tsukamoto et al., 2000). We generated an expression construct encoding a fluorescent bait protein consisting of a fluorescent protein (FP), the lac repressor (LacI) and the protein X to be tested for interactions (bait) resulting in the triple fusion protein *FP-LacI-X* (Fig. 1 a) or *X-LacI-*FP. This fusion protein binds to the operator array, which then becomes visible due to the focal enrichment of the FP signal. A second, differently labeled fusion protein (FP-Y, prey) may either interact with the bait protein X leading to co-localization of the FP signals (Fig. 1 b) or may not interact, resulting in a dispersed distribution of the prey fluorescence (Fig. 1 c).

### Example 3: Visualization of interactions between DNA repair proteins

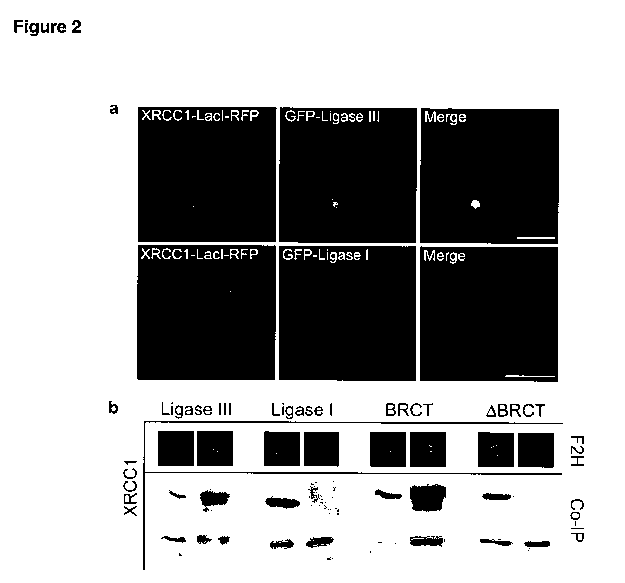

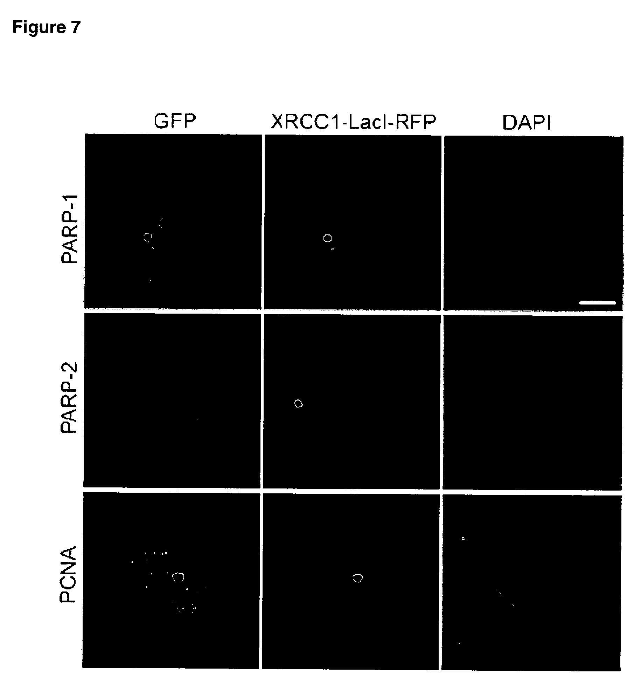

To test the F2H assay, the previously described interaction between the two DNA repair proteins DNA Ligase III and XRCC1 (Caldecott et al., 1994; Wei et al., 1995) was analyzed and the results were compared with data obtained from pull down assays. We have previously shown that this interaction is mediated by the BRCT domain of DNA Ligase III which targets it to DNA repair sites (Mortusewicz et al., 2006). We generated a bait fusion protein consisting of XRCC1 followed by the LacI and the monomeric red fluorescent protein RFP (mRFP). As expected this fusion protein localized at the *lac* operator array in transiently transfected BHK cells (Fig. 2a). Both, the full length GFP-tagged DNA Ligase III as well as the isolated GFP-labeled BRCT domain co-localize with XRCC1 at the *lac* operator array, while a fusion protein missing the BRCT domain shows a dispersed distribution. Notably, the highly homologous DNA Ligase I, which catalyzes the same reaction as DNA Ligase III, does not bind to XRCC1 (Fig. 2a and Figure 6). A direct comparison of the F2H data with data obtained from Co-IP experiments reveals that these two methods gave similar results (Fig. 2b). In addition, we could also observe the recently described interaction of XRCC1 with PCNA (Fan et al., 2004) and the two DNA-damage dependent PARPs, PARP-1 and PARP-2 (Masson et al., 1998; Schreiber et al., 2002) (Figure 7). These results demonstrate that the F2H assay is well suited to study protein-protein interactions in living cells.

### Example 4: Analysis of cell cycle dependence of protein-protein interactions

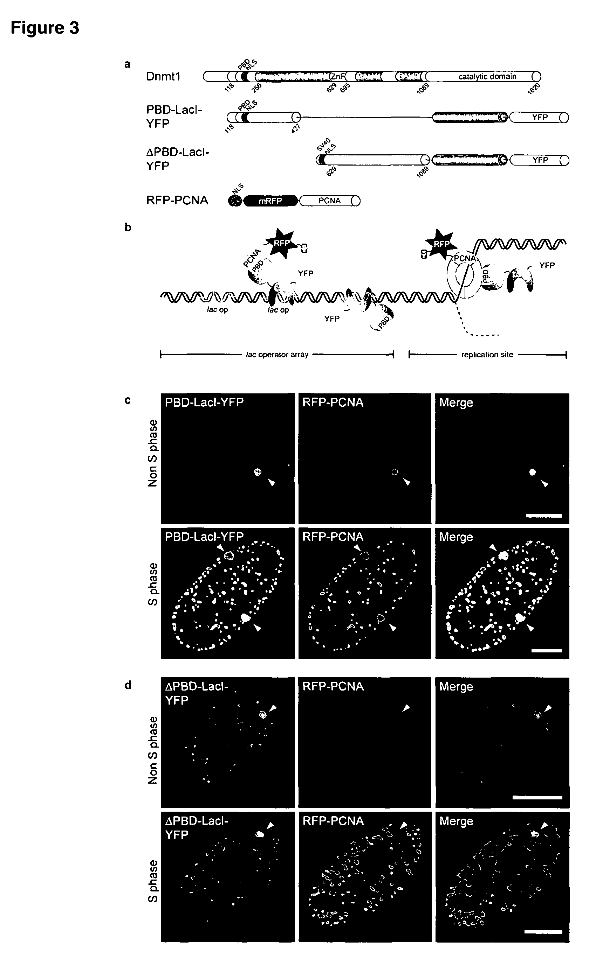

A challenge in the analysis of protein-protein interactions is to monitor transient changes caused by for example cell cycle progression or other external stimuli. We analyzed the previously described interaction between DNA methyltransferase 1 (Dnmt1) and PCNA which is mediated by the PCNA binding domain (PBD) and targets Dnmt1 to sites of DNA replication in S phase (Chuang et al., 1997; Easwaran et al., 2004). These findings raised the question whether this interaction occurs only in S phase at replication foci or throughout the cell cycle. We generated two bait-proteins comprising parts of Dnmt1 fused to the LacI and YFP. One bait (PBD-Lacl-YFP) comprises aa 118-427 of Dnmt1 including the PBD, while the second bait (ΔPBD-LacI-YFP) lacks the PBD and comprises aa 629-1089 of Dnmt1 (**Fig. 3a**). As a prey-protein we used RFP-PCNA which in addition marks sites of DNA replication allowing the identification of cells in S phase (Easwaran et al., 2005; Sporbert et al., 2005). The binding possibilities of these fusion proteins at the *lac* operator array and the replication fork are summarized in Fig. 3b.

In non S phase the LacI part of the bait proteins only binds to the chromosomally integrated *lac* operator array, which - dependent on the ploidy of the cell - becomes visible as one or two fluorescent spots in the nucleus. Interaction of RFP-PCNA with the PBD part of the bait protein results in co-localization of the fluorescent signals at the *lac* operator array (Fig. 3c upper panel), while deletion of the PBD in the bait protein leads to a dispersed distribution of RFP-PCNA in non S phase cells (Fig. 3d upper panel). This clearly illustrates that the PBD-dependent interaction of Dnmt1 with PCNA also occurs outside of S phase.

In S phase cells, RFP-PCNA localizes at sites of ongoing DNA replication and in addition is recruited to the *lac* operator array by the PBD-Lacl-YFP bait protein (Fig. 3c lower panel). In contrast, when RFP-PCNA is coexpressed together with a bait protein lacking a functional PBD (ΔPBD-LacI-YFP), RFP-PCNA is exclusively enriched at DNA replication sites and not at the *lac* operator array highlighted by ΔPBD-LacI-YFP (Fig. 3d lower panel).

These results clearly show that the localization of RFP-PCNA (prey) at the *lac* operator array depends on the presence of the PBD in the bait construct and that this interaction is not restricted to S phase.

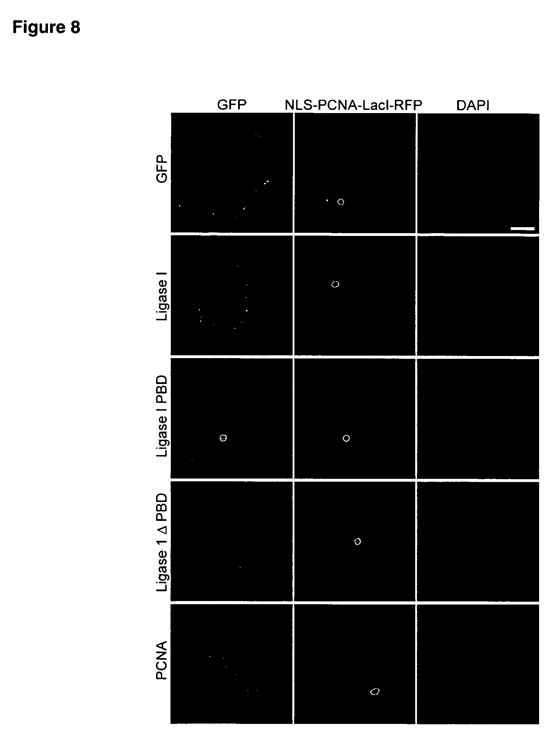

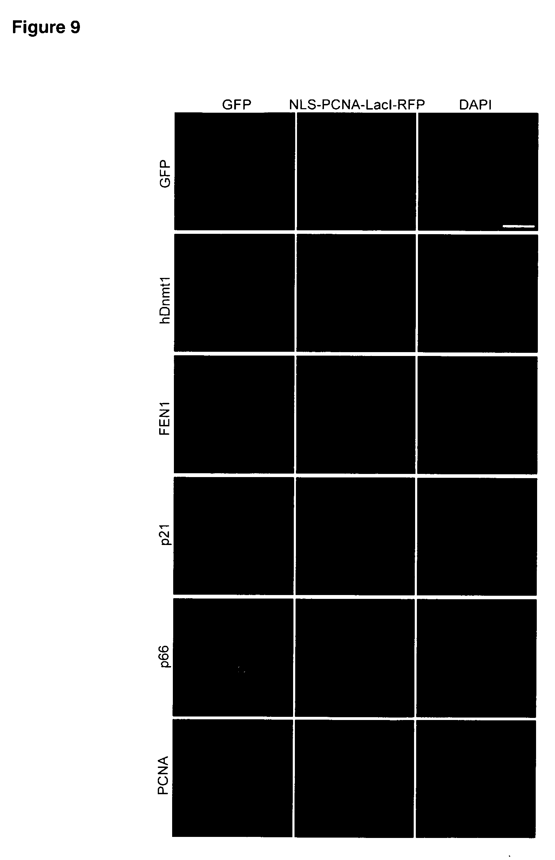

Next we analyzed the interaction of other PBD-containing proteins with PCNA. We generated a bait fusion protein comprising PCNA fused to an additional NLS followed by LacI and RFP (NLS-PCNA-Lacl-RFP). When co-expressed with GFP-Ligase I, both fusion proteins localized to the *lac* operator array indicating interaction between PCNA and DNA Ligase I. Deletion of the PBD lead to a disperse distribution of DNA Ligase I, while the PBD of DNA Ligase I alone was sufficient for binding to PCNA at the *lac* operator array (Figure 8). This is in agreement with previous studies showing that the PBD of DNA Ligase I is necessary and sufficient for its targeting to DNA replication and repair sites (Cardoso et al., 1997; Montecucco et al., 1995; Mortusewicz et al., 2006). Notably, using the F2H assay we could demonstrate that DNA Ligase I, as well as the isolated PBD are capable of binding to PCNA also outside of S-phase. Likewise we could show binding of various additional replication and repair proteins like FEN1, p21 and the Polymerase δ subunit p66 to PCNA in non S-phase cells (Figure 9). Taken together we could show that the interaction between replication proteins and PCNA is not limited to S phase but also occurs in non S phase cells and outside the replication machinery.

This illustrates that the F2H assay offers the unique potential to analyze cell cycle specific changes in protein-protein interactions in living cells.

### Example 5: Detection of interactions between proteins related to Huntington's disease

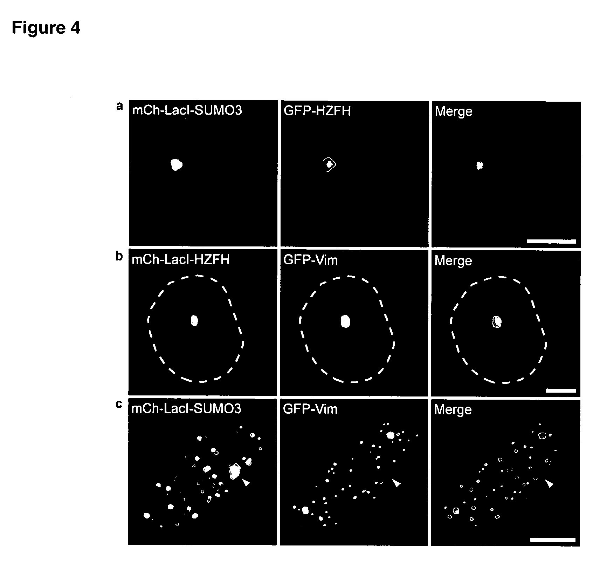

To investigate whether the F2H assay can also detect protein-protein interactions taking place in other cellular compartments, we tested the F2H assay with protein interactions identified in the context of Huntington's disease by yeast two-hybrid (Y2H) assays (Goehler et al., 2004). We analyzed the interaction of one cytoplasmatic (Vimentin) and two nuclear (HZFH and SUMO3) proteins. Vimentin has been described to be a cytoskeleton component and participates in transport processes, whereas HZFH and SUMO3 are involved in transcriptional regulation and DNA maintenance (Goehler et al., 2004). These proteins were either fused with a red fluorescent mCherry-LacI-NLS or with NLS-GFP to generate sets of bait and prey proteins. BHK cells carrying a *lac* operator array were transfected with all possible combinations of expression constructs and subjected to microscopic analysis. We could detect an interaction between Vimentin and HZFH independent of whether these two proteins were used as bait or prey (Fig.4 and data not shown). We could also detect the reported interaction between SUMO3 and HZFH while Vimentin and SUMO3 did not interact, as previously described (Fig. 4) (Goehler et al., 2004). These results show that interactions of nuclear and cytoplasmic proteins can be studied with the F2H assay.

### Example 6: Detection of interactions between mitochondrial proteins

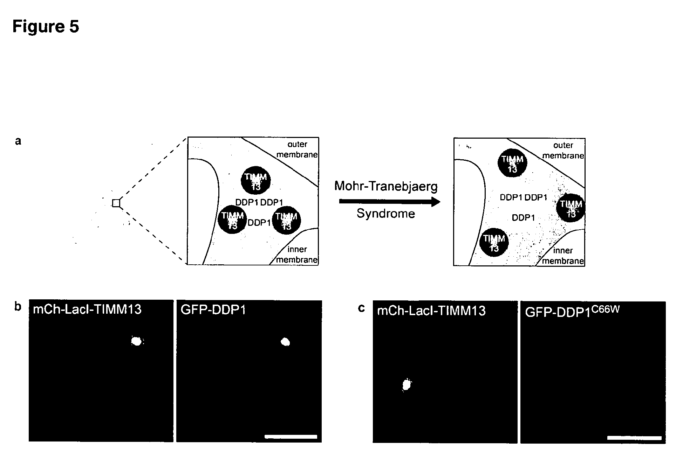

Next, we investigated whether the F2H assay is also suitable to detect protein-protein interactions occurring in other cellular organelles. To this end, we analyzed the interaction between two mitochondrial proteins, DDP1 (deafness dystonia peptide 1) and TIMM13. Both proteins are nuclear encoded and imported into the mitochondrial intermembrane space (IMS) forming a hexameric complex (Fig. 5a). Within the IMS the DDP1-TIMM13 complex facilitates the import of hydrophobic proteins of the mitochondrial import machinery into the mitochondrial innermembrane (Rothbauer et al., 2001). A mutation of the *DDP1* gene was associated with the Mohr-Tranebjaerg-Syndrome, which is a progressive, neurodegenerative disorder (Tranebjaerg et al., 1995). This C66W missense mutation is known to cause a full blown phenotype and affects the highly conserved Cys(4) motif of DDP1. Previous studies have shown, that this amino acid exchange abolishes the interaction between DDP1 and TIMM13 in the IMS (Hofmann et al., 2002).

Using a red fluorescent bait fusion protein comprising Lacl-NLS-TIMM13 and GFP-tagged wildtype (GFP-DDP1) or mutant DDP1 (GFP-DDP1^{C66W}) prey proteins we analyzed this specific mitochondrial protein interaction with the F2H assay. We found that GFP-DDP1 co-localizes with TIMM13 at the *lac* operator array (Fig. 5b), while GFP-DDP1^{C66W} was evenly distributed (Fig. 5c). These results demonstrate that the F2H assay is also suitable for the analysis of protein-protein interactions occurring outside the nucleus and the characterization of disease related point mutations disrupting these interactions.

### References

Ando, R., H. Hama, M. Yamamoto-Hino, H. Mizuno, and A. Miyawaki. 2002. An optical marker based on the UV-induced green-to-red photoconversion of a fluorescent protein. Proc Natl Acad Sci U S A. 99:12651-6.
Baird, G.S., D.A. Zacharias, and R.Y. Tsien. 2000. Biochemistry, mutagenesis, and oligomerization of DsRed, a red fluorescent protein from coral. Proc Natl Acad Sci U S A. 97:11984-9.
Bebbington, C.R., G. Renner, S. Thomson, D. King, D. Abrams, and G.T. Yarranton. 1992. High-level expression of a recombinant antibody from myeloma cells using a glutamine synthetase gene as an amplifiable selectable marker. Biotechnology (N Y). 10:169-75.
Beddoe, T., M. Ling, S. Dove, O. Hoegh-Guldberg, R.J. Devenish, M. Prescott, and J. Rossjohn. 2003. The production, purification and crystallization of a pocilloporin pigment from a reef-forming coral. Acta Crystallogr D Biol Crystallogr. 59:597-9.
Borden, K.L. 2002. Pondering the promyelocytic leukemia protein (PML) puzzle: possible functions for PML nuclear bodies. Mol Cell Biol. 22:5259-69.
Braasch, D.A., and D.R. Corey. 2001. Locked nucleic acid (LNA): fine-tuning the recognition of DNA and RNA. Chem Biol. 8:1-7.
Bulina, M.E., D.M. Chudakov, N.N. Mudrik, and K.A. Lukyanov. 2002. Interconversion of Anthozoa GFP-like fluorescent and non-fluorescent proteins by mutagenesis. BMC Biochem. 3:7*.*
Bunch, T.A., Y. Grinblat, and L.S. Goldstein. 1988. Characterization and use of the Drosophila metallothionein promoter in cultured Drosophila melanogaster cells. Nucleic Acids Res. 16:1043-61.
Caldecott, K.W., C.K. McKeown, J.D. Tucker, S. Ljungquist, and L.H. Thompson. 1994. An interaction between the mammalian DNA repair protein XRCC1 and DNA ligase III. Mol Cell Biol. 14:68-76.
Campbell, R.E., O. Tour, A.E. Palmer, P.A. Steinbach, G.S. Baird, D.A. Zacharias, and R.Y. Tsien. 2002. A monomeric red fluorescent protein. Proc Natl Acad Sci U S A. 99:7877-82.
Cardoso, M.C., C. Joseph, H.P. Rahn, R. Reusch, B. Nadal-Ginard, and H. Leonhardt. 1997. Mapping and use of a sequence that targets DNA ligase I to sites of DNA replication in vivo. J Cell Biol. 139:579-87.
Cazzalini, O., P. Perucca, F. Riva, L.A. Stivala, L. Bianchi, V. Vannini, B. Ducommun, and E. Prosperi. 2003. p21CDKN1A does not interfere with loading of PCNA at DNA replication sites, but inhibits subsequent binding of DNA polymerase delta at the G1/S phase transition. Cell Cycle. 2:596-603.
Chuang, L.S., H.I. Ian, T.W. Koh, H.H. Ng, G. Xu, and B.F. Li. 1997. Human DNA-(cytosine-5) methyltransfe rase- PC NA complex as a target for p21WAF1. Science. 277:1996-2000.
Dietzel, S., K. Zolghadr, C. Hepperger, and A.S. Belmont. 2004. Differential large-scale chromatin compaction and intranuclear positioning of transcribed versus non-transcribed transgene arrays containing {beta}-globin regulatory sequences. J Cell Sci. 117:4603-4614.
Dove, S.G., O. Hoegh-Guldberg, and S. Ranganathan. 2001. Major colour patterns of reef-building corals are due to a family of GFP-like proteins. Coral Reefs. 19:197-204.
Easwaran, H.P., H. Leonhardt, and M.C. Cardoso. 2005. Cell Cycle Markers for Live Cell Analyses. Cell Cycle. 4*.*
Easwaran, H.P., L. Schermelleh, H. Leonhardt, and M.C. Cardoso. 2004. Replication-independent chromatin loading of Dnmt1 during G2 and M phases. EMBO Rep. 5:1181-6.
Fan, J., M. Otterlei, H.K. Wong, A.E. Tomkinson, and D.M. Wilson, 3rd. 2004. XRCC1 co-localizes and physically interacts with PCNA. Nucleic Acids Res. 32:2193-201.
Goehler, H., M. Lalowski, U. Stelzl, S. Waelter, M. Stroedicke, U. Worm, A. Droege, K.S. Lindenberg, M. Knoblich, C. Haenig, M. Herbst, J. Suopanki, E. Scherzinger, C. Abraham, B. Bauer, R. Hasenbank, A. Fritzsche, A.H. Ludewig, K. Bussow, S.H. Coleman, C.A. Gutekunst, B.G. Landwehrmeyer, H. Lehrach, and E.E. Wanker. 2004. A protein interaction network links GIT1, an enhancer of huntingtin aggregation, to Huntington's disease. Mol Cell. 15:853-65.
Gurskaya, N.G., A.F. Fradkov, A. Terskikh, M.V. Matz, Y.A. Labas, V.I. Martynov, Y.G. Yanushevich, K.A. Lukyanov, and S.A. Lukyanov. 2001. GFP-like chromoproteins as a source of far-red fluorescent proteins. FEBS Lett. 507:16-20.
Hofmann, S., U. Rothbauer, N. Muhlenbein, W. Neupert, K.D. Gerbitz, M. Brunner, and M.F. Bauer. 2002. The C66W mutation in the deafness dystonia peptide 1 (DDP1) affects the formation of functional DDP1.TIM13 complexes in the mitochondrial intermembrane space. J Biol Chem. 277:23287-93.
Horton, R.M., H.D. Hunt, S.N. Ho, J.K. Pullen, and L.R. Pease. 1989. Engineering hybrid genes without the use of restriction enzymes: gene splicing by overlap extension. Gene. 77:61-8.
Janicki, S.M., T. Tsukamoto, S.E. Salghetti, W.P. Tansey, R. Sachidanandam, K.V. Prasanth, T. Ried, Y. Shav-Tal, E. Bertrand, R.H. Singer, and D.L. Spector. 2004. From silencing to gene expression: real-time analysis in single cells. Cell. 116:683-98.
Kalderon, D., W.D. Richardson, A.F. Markham, and A.E. Smith. 1984. Sequence requirements for nuclear location of simian virus 40 large-T antigen. Nature. 311:33-8.
Kerppola, T.K. 2006. Visualization of molecular interactions by fluorescence complementation. Nat Rev Mol Cell Biol. 7:449-56.
Lukyanov, K.A., A.F. Fradkov, N.G. Gurskaya, M.V. Matz, Y.A. Labas, A.P. Savitsky, M.L. Markelov, A.G. Zaraisky, X. Zhao, Y. Fang, W. Tan, and S.A. Lukyanov. 2000. Natural animal coloration can Be determined by a nonfluorescent green fluorescent protein homolog. J Biol Chem. 275:25879-82.
Maeda, Y., T.C. Hunter, D.E. Loudy, V. Dave, V. Schreiber, and J.A. Whitsett. 2006. PARP-2 interacts with TTF-1 and regulates expression of surfactant protein-B. J Biol Chem. 281:9600-6.
Masson, M., C. Niedergang, V. Schreiber, S. Muller, J. Menissier-de Murcia, and G. de Murcia. 1998. XRCC1 is specifically associated with poly(ADP-ribose) polymerase and negatively regulates its activity following DNA damage. Mol Cell Biol. 18:3563-71.
Matz, M.V., A.F. Fradkov, Y.A. Labas, A.P. Savitsky, A.G. Zaraisky, M.L. Markelov, and S.A. Lukyanov. 1999. Fluorescent proteins from nonbioluminescent Anthozoa species. Nat Biotechnol. 17:969-73.
Maul, G.G., D. Negorev, P. Bell, and A.M. Ishov. 2000. Review: properties and assembly mechanisms of ND10, PML bodies, or PODs. J Struct Biol. 129:278-87.
Meder, V.S., M. Boeglin, G. de Murcia, and V. Schreiber. 2005. PARP-1 and PARP-2 interact with nucleophosmin/B23 and accumulate in transcriptionally active nucleoli. J Cell Sci. 118:211-22.
Merzlyak, E.M., J. Goedhart, D. Shcherbo, M.E. Bulina, A.S. Shcheglov, A.F. Fradkov, A. Gaintzeva, K.A. Lukyanov, S. Lukyanov, T.W. Gadella, and D.M. Chudakov. 2007. Bright monomeric red fluorescent protein with an extended fluorescence lifetime. Nat Methods. 4:555-7.
Miller, C.L., M.M. Arnold, T.J. Broering, C. Eichwald, J. Kim, J.B. Dinoso, and M.L. Nibert. 2007. Virus-derived platforms for visualizing protein associations inside cells. Mol Cell Proteomics. 6:1027-38.
Miyawaki, A. 2003. Visualization of the spatial and temporal dynamics of intracellular signaling. Dev Cell. 4:295-305.
Moller, A., H. Sirma, T.G. Hofmann, S. Rueffer, E. Klimczak, W. Droge, H. Will, and M.L. Schmitz. 2003. PML is required for homeodomain-interacting protein kinase 2 (HIPK2)-mediated p53 phosphorylation and cell cycle arrest but is dispensable for the formation of HIPK domains. Cancer Res. 63:4310-4.
Montecucco, A., E. Savini, F. Weighardt, R. Rossi, G. Ciarrocchi, A. Villa, and G. Biamonti. 1995. The N-terminal domain of human DNA ligase I contains the nuclear localization signal and directs the enzyme to sites of DNA replication. Embo J. 14:5379-86.
Mortusewicz, O., U. Rothbauer, M.C. Cardoso, and H. Leonhardt. 2006. Differential recruitment of DNA Ligase I and III to DNA repair sites. Nucleic Acids Res. 34:3523-32.
Mortusewicz, O., L. Schermelleh, J. Walter, M.C. Cardoso, and H. Leonhardt. 2005. Recruitment of DNA methyltransferase I to DNA repair sites. Proc Natl Acad Sci U S A.
Murphy, G., M.I. Cockett, R.V. Ward, and A.J. Docherty. 1991. Matrix metalloproteinase degradation of elastin, type IV collagen and proteoglycan. A quantitative comparison of the activities of 95 kDa and 72 kDa gelatinases, stromelysins-1 and -2 and punctuated metalloproteinase (PUMP). Biochem J. 277 ( Pt 1):277-9.
Newman, J.R., S. Ghaemmaghami, J. Ihmels, D.K. Breslow, M. Noble, J.L. DeRisi, and J.S. Weissman. 2006. Single-cell proteomic analysis of S. cerevisiae reveals the architecture of biological noise. Nature. 441:840-6.
Parrish, J.R., K.D. Gulyas, and R.L. Finley, Jr. 2006. Yeast two-hybrid contributions to interactome mapping. Curr Opin Biotechnol. 17:387-93.
Phair, R.D., and T. Misteli. 2000. High mobility of proteins in the mammalian cell nucleus. Nature. 404:604-9.
Robinett, C.C., A. Straight, G. Li, C. Willhelm, G. Sudlow, A. Murray, and A.S. Belmont. 1996. In vivo localization of DNA sequences and visualization of large-scale chromatin organization using lac operator/repressor recognition. J Cell Biol. 135:1685-700.
Rothbauer, U., S. Hofmann, N. Muhlenbein, S.A. Paschen, K.D. Gerbitz, W. Neupert, M. Brunner, and M.F. Bauer. 2001. Role of the deafness dystonia peptide 1 (DDP1) in import of human Tim23 into the inner membrane of mitochondria. J Biol Chem. 276:37327-34.
Rothbauer, U., K. Zolghadr, S. Muyldermans, A. Schepers, M.C. Cardoso, and H. Leonhardt. 2007. A versatile nanotrap for biochemical and functional studies with fluorescent fusion proteins. Mol Cell Proteomics*.*
Schermelleh, L., F. Spada, H.P. Easwaran, K. Zolghadr, J.B. Margot, M.C. Cardoso, and H. Leonhardt. 2005. Trapped in action: direct visualization of DNA methyltransferase activity in living cells. Nat Methods. 2:751-756.
Schreiber, V., J.C. Ame, P. Dolle, I. Schultz, B. Rinaldi, V. Fraulob, J. Menissier-de Murcia, and G. de Murcia. 2002. Poly(ADP-ribose) polymerase-2 (PARP-2) is required for efficient base excision DNA repair in association with PARP-1 and XRCC1. J Biol Chem. 277:23028-36.
Sekar, R.B., and A. Periasamy. 2003. Fluorescence resonance energy transfer (FRET) microscopy imaging of live cell protein localizations. J Cell Biol. 160:629-33.
Shaner, N.C., R.E. Campbell, P.A. Steinbach, B.N. Giepmans, A.E. Palmer, and R.Y. Tsien. 2004. Improved monomeric red, orange and yellow fluorescent proteins derived from Discosoma sp. red fluorescent protein. Nat Biotechnol. 22:1567-72.
Sporbert, A., P. Domaing, H. Leonhardt, and M.C. Cardoso. 2005. PCNA acts as a stationary loading platform for transiently interacting Okazaki fragment maturation proteins. Nucleic Acids Res. 33:3521-8.
Terskikh, A., A. Fradkov, G. Ermakova, A. Zaraisky, P. Tan, A.V. Kajava, X. Zhao, S. Lukyanov, M. Matz, S. Kim, I. Weissman, and P. Siebert. 2000. "Fluorescent timer": protein that changes color with time. Science. 290:1585-8.
Tranebjaerg, L., C. Schwartz, H. Eriksen, S. Andreasson, V. Ponjavic, A. Dahl, R.E. Stevenson, M. May, F. Arena, D. Barker, and et al. 1995. A new X linked recessive deafness syndrome with blindness, dystonia, fractures, and mental deficiency is linked to Xq22. J Med Genet. 32:257-63.
Tsien, R.Y. 1998. The green fluorescent protein. Annu Rev Biochem. 67:509-44.
Tsukamoto, T., N. Hashiguchi, S.M. Janicki, T. Tumbar, A.S. Belmont, and D.L. Spector. 2000. Visualization of gene activity in living cells. Nat Cell Biol. 2:871-8.
Tu, H., Q. Xiong, S. Zhen, X. Zhong, L. Peng, H. Chen, X. Jiang, W. Liu, W. Yang, J. Wei, M. Dong, W. Wu, and A. Xu. 2003. A naturally enhanced green fluorescent protein from magnificent sea anemone (Heteractis magnifica) and its functional analysis. Biochem Biophys Res Commun. 301:879-85.
Tumbar, T., G. Sudlow, and A.S. Belmont. 1999. Large-scale chromatin unfolding and remodeling induced by VP16 acidic activation domain. J Cell Biol. 145:1341-54.
Vazquez, J., A.S. Belmont, and J.W. Sedat. 2001. Multiple regimes of constrained chromosome motion are regulated in the interphase Drosophila nucleus. Curr Biol. 11:1227-39.
Wei, Y.F., P. Robins, K. Carter, K. Caldecott, D.J. Pappin, G.L. Yu, R.P. Wang, B.K. Shell, R.A. Nash, P. Schar, and et al. 1995. Molecular cloning and expression of human cDNAs encoding a novel DNA ligase IV and DNA ligase III, an enzyme active in DNA repair and recombination. Mol Cell Biol. 15:3206-16.
Wiedenmann, J., C. Elke, K.D. Spindler, and W. Funke. 2000. Cracks in the beta-can: fluorescent proteins from Anemonia sulcata (Anthozoa, Actinaria). Proc Natl Acad Sci U S A. 97:14091-6.
Wiehler, J., J. von Hummel, and B. Steipe. 2001. Mutants of Discosoma red fluorescent protein with a GFP-like chromophore. FEBS Lett. 487:384-9.
Zhang, J., R.E. Campbell, A.Y. Ting, and R.Y. Tsien. 2002. Creating new fluorescent probes for cell biology. Nat Rev Mol Cell Biol. 3:906-18.
Zimmer, M. 2002. Green fluorescent protein (GFP): applications, structure, and related photophysical behavior. Chem Rev. 102:759-81.

## Claims

1. An in vitro method for detecting protein-protein interactions comprising:
(a) expressing in a eukaryotic cell a first fusion protein comprising
(i) a (poly)peptide that, when expressed in a cell, accumulates at distinct sites in the nucleus of the cell; and
(ii) a (poly)peptide specifically binding to green fluorescent protein (GFP)
(b) expressing in the same cell a second fusion protein comprising
(i) GFP; and
(ii) a bait (poly)peptide
(c) expressing in the same cell a third fusion protein comprising
(i) a fluorescent (poly)peptide, the excitation and/or emission wavelength of which differs from that of GFP; and
(ii) a prey (poly)peptide
(d) detecting the fluorescence emission of the fluorescent parts of the second and the third fusion protein in the cell upon excitation,
wherein a co-localization of the fluorescence emission of both fusion proteins in the cell nucleus is indicative of an interaction of the bait and the prey (poly)peptide.

2. An in vitro method for identifying a compound modulating the interaction of two (poly)peptides
(a) expressing in a eukaryotic cell a first fusion protein comprising
(i) a (poly)peptide that, when expressed in a cell, accumulates at distinct sites in the nucleus of the cell;and
(ii) a (poly)peptide specifically binding to green fluorescent protein (GFP)
(b) expressing in the same cell a second fusion protein comprising
(i) GFP; and
(ii) a bait (poly)peptide
(c) expressing in the same cell a third fusion protein comprising
(i) a fluorescent (poly)peptide, the excitation and/or emission wavelength of which differs from that of GFP; and
(ii) a prey (poly)peptide known or suspected to interact with the bait (poly)peptide
(d) contacting the cell with a test compound; and
(e) detecting the fluorescence emission of the fluorescent parts of the second and the third fusion protein in the cell upon excitation;
wherein a change in the degree of co-localization of the fluorescence emission of the fluorescent parts of the second and the third fusion protein in the cell nucleus as compared to that observed in the nucleus of a reference cell not contacted with the test compound is indicative that the compound is capable of modulating the interaction of the bait and the prey (poly)peptide.

3. An in vitro method of determining the relative strength of the interaction of two proteins with a third protein comprising:
(a) expressing in a eukaryotic cell a first fusion protein comprising
(i) a (poly)peptide that, when expressed in a cell, accumulates at distinct sites in the nucleus of the cell; and
(ii) a (poly)peptide specifically binding to green fluorescent protein (GFP)
(b) expressing in the same cell a second fusion protein comprising
(i) GFP; and
(ii) a bait (poly)peptide
(c) expressing in the same cell a third fusion protein comprising
(i) a fluorescent (poly)peptide, the excitation and/or emission wavelength of which differs from that of GFP; and
(ii) a first prey (poly)peptide
(d) expressing in the same cell a fourth fusion protein comprising
(i) a fluorescent (poly)peptide, the excitation and/or emission wavelength of which differs from that of GFP and from that of the fluorescent (poly)peptide of said third fusion protein; and
(ii) a second prey (poly)peptide
(e) detecting the fluorescence emission of the fluorescent parts of the second and the third fusion protein in the cell upon excitation,
wherein an extent of co-localization of the fluorescence emission of the second and the third fusion protein different as compared to that of the second and the fourth fusion protein in the cell nucleus is indicative of a different binding strength of the first and the second prey (poly)peptide to the bait (poly)peptide.

4. An in vitro method for detecting protein-protein interactions comprising:
(a) expressing in a eukaryotic cell a first fusion protein comprising
(i) a fluorescent (poly)peptide;
(ii) a (poly)peptide that, when expressed in a cell, accumulates at distinct sites in the nucleus of the cell; and
(iii) a bait (poly)peptide;
(b) expressing in the same cell a second fusion protein comprising
(i) a fluorescent (poly)peptide, the excitation and/or emission wavelength of which differs from that of the fluorescent (poly)peptide comprised in said first fusion protein; and
(ii) a prey (poly)peptide;
(c) detecting the fluorescence emission of the fluorescent parts of the first and the second fusion protein in the cell upon excitation,
wherein a co-localization of the fluorescence emission of both fusion proteins in the cell nucleus is indicative of an interaction of the bait and the prey (poly)peptide.

5. An in vitro method for identifying a compound modulating the interaction of two (poly)peptides
(a) expressing in a eukaryotic cell a first fusion protein comprising
(i) a fluorescent (poly)peptide
(ii) a (poly)peptide that, when expressed in a cell, accumulates at distinct sites in the nucleus of the cell; and
(iii) a bait (poly)peptide
(b) expressing in the same cell a second fusion protein comprising
(i) a fluorescent (poly)peptide, the excitation and/or emission wavelength of which differs from that of the fluorescent (poly)peptide comprised in said first fusion protein; and
(ii) a prey (poly)peptide known or suspected to interact with the bait (poly)peptide
(c) contacting the cell with a test compound; and
(d) detecting the fluorescence emission of the fluorescent parts of the first and the second fusion protein in the cell upon excitation;
wherein a change in the degree of co-localization of the fluorescence emission of the fluorescent parts of both fusion proteins in the cell nucleus as compared to that observed in the nucleus of a reference cell not contacted with the test compound is indicative that the compound is capable of modulating the interaction of the bait and the prey (poly)peptide.

6. An in vitro method of determining the relative strength of the interaction of two proteins with a third protein comprising
(a) expressing in a eukaryotic cell a first fusion protein comprising
(i) a fluorescent (poly)peptide;
(ii) a (poly)peptide that, when expressed in a cell, accumulates at distinct sites in the nucleus of the cell; and
(iii) a bait (poly)peptide
(b) expressing in the same cell a second fusion protein comprising
(i) a fluorescent (poly)peptide, the excitation and/or emission wavelength of which differs from that of the fluorescent (poly)peptide comprised in said first fusion protein; and
(ii) a first prey (poly)peptide
(c) expressing in the same cell a third fusion protein comprising
(i) a fluorescent (poly)peptide, the excitation and/or emission wavelength of which differs from that of the fluorescent (poly)peptide comprised in said first and second fusion protein; and
(ii) a second prey (poly)peptide
(d) detecting the fluorescence emission of the fluorescent parts of the first, the second and the third fusion protein in the cell upon excitation,
wherein an extent of co-localization of the fluorescence emission of the first and the second fusion protein different as compared to that of the first and the third fusion protein in the cell nucleus is indicative of a different binding strength of the first and the second prey protein to the bait (poly)peptide.

7. The method of claim 2 or 5, wherein a decrease of co-localization of the fluorescent parts of both fusion proteins in the cell nucleus as compared to that observed in the nucleus of a reference cell not contacted with the test compound is indicative of the compound being capable of weakening the interaction of the bait and the prey (poly)peptide.

8. The method of claim 2 or 5, wherein an increase in co-localization of the fluorescent parts of both fusion proteins in the cell nucleus as compared to that observed in the nucleus of a reference cell not contacted with the test compound is indicative of the compound being capable of inducing or enhancing the interaction of the bait and the prey (poly)peptide.

9. The method of claim 4, wherein the detection is employed for investigating the dependency of protein-protein interactions on cellular processes and wherein the method further comprises (d1) monitoring the fluorescence emission of the fluorescent parts of the first and the second fusion protein in the cell in the course of one or more processes in the cell; wherein a change in the degree of co-localization of the fluorescence emission of the fluorescent parts of both fusion proteins in the cell is indicative of a dependency of the interaction on the one or more cellular processes.

10. The method of claim 1, wherein the detection is employed for investigating the dependency of
protein-protein interactions on cellular processes and wherein the method further comprises (d1) monitoring the fluorescence emission of the fluorescent parts of the second and the third fusion protein in the cell in the course of one or more processes in the cell; wherein a change in the degree of co-localization of the fluorescence emission of the fluorescent parts of both fusion proteins in the cell is indicative of a dependency of the interaction on the one or more cellular processes.

11. The method of claim 9 or 10, wherein the cellular process is the cell cycle, secretion, translocation or signal transduction.

12. The method of claim 4, wherein the detection is employed for determining the strength of a protein-protein interaction and wherein the method further comprises in case that a co-localization of the fluorescence emission of the fluorescent parts of both fusion proteins is detected (d2) selective extinction of the fluorescence of said second fusion protein and monitoring the restoration of co-localization of the fluorescence emission of the fluorescent parts of both fusion proteins over time, wherein the time needed to establish co-localization is indicative of the strength of the protein-protein interaction.

13. The method of claim 1, wherein the detection is employed for determining the strength of a protein-protein interaction and wherein the method further comprises in case that a co-localization of the fluorescence emission of the fluorescent parts of both fusion proteins is detected (d2) selective extinction of the fluorescence of said third fusion protein and monitoring the restoration of co-localization of the fluorescence emission of the fluorescent parts of both fusion proteins over time, wherein the time needed to establish co-localization is indicative of the strength of the protein-protein interaction.

14. The method of any one of claims 1 to 13 wherein the (poly)peptide that, when expressed in a cell, accumulates at distinct sites in the nucleus of the cell directly interacts with proteinaceous or non-proteinaceous structures accumulated at distinct sites in the nucleus of the cell.

15. The method of any one of claims 1 to 13 wherein the (poly)peptide that, when expressed in a cell, accumulates at distinct sites in the nucleus of the cell indirectly interacts with proteinaceous or non-proteinaceous structures accumulated at distinct sites in the nucleus of the cell.

16. The method of any one of claims 1 to 13, wherein components (i), (ii) and/or (iii) of said first fusion protein and/or components (i) and (ii) of said second fusion protein and/or components (i) and (ii) of said third fusion protein and/or components (i) and (ii) of said fourth fusion protein are connected via a linker.

17. The method of any one of claims 1 to 16, wherein any of the prey (poly)peptides comprises a nuclear localization signal.

18. The method of any one of claims 1 to 3, wherein said second and/or third and/or fourth fusion protein comprises a nuclear localization signal.

19. The method of any one of claims 1 to 18, wherein expression in the eukaryotic cell is effected by transfecting the nucleic acid molecules encoding said first and second fusion protein in one or more vectors.

20. The method of any one of claims 1 to 19, wherein the (poly)peptide accumulated at distinct sites of the nucleus of the cell has been introduced into the cell.

21. The method of any one of claims 1 to 20, wherein the distinct sites of the cell form inert structures.

22. The method of claim 21, wherein the inert structure is the nuclear lamina or nuclear specles.

23. The method of any one of claims 1 to 20, wherein the (poly)peptide that, when expressed in a cell, accumulates at distinct sites in the nucleus of the cell interacts with PML bodies.

24. The method of any one of claims 1 to 23, wherein the (poly)peptide accumulated at distinct sites of the nucleus is heterologous to the cell.

25. The method of any one of claims 1 to 21 and 24, wherein the (poly)peptide that, when expressed in a cell, accumulates at distinct sites in the nucleus of the cell interacts with DNA.

26. The method of claim 25, wherein the DNA is the lac-operator.

27. The method of claim 26, wherein the lac-operator is present in the nucleus in multiple copies.

28. The method of any one of claims 1 to 27, wherein said first fusion protein comprises Lacl.

29. The method of any one of claims 1 to 28, wherein the detection is carried out using a fluorescence microscope.

30. The method of any one of claims 1 to 29, wherein the eukaryotic cell is a living cell.

31. The method of any one of claims 1 to 30 further comprising localizing the bait and/or prey (poly)peptides in the cell.

32. A vector for use in expressing the first and second fusion protein according to the method of any one of claims 4 to 6 in a eukaryotic cell comprising
A. a nucleic acid molecule encoding the fusion protein comprising
(i) a fluorescent (poly)peptide;
(ii) a (poly)peptide that, when expressed in a cell, accumulates at distinct sites in the nucleus of the cell; and
(iii) a bait (poly)peptide; and
B. a nucleic acid molecule encoding the fusion protein comprising
(i) a fluorescent (poly)peptide, the excitation and/or emission wavelength of which differs from that of the fluorescent (poly)peptide comprised in the fusion protein encoded by the nucleic acid molecule according to A; and
(ii) a prey (poly)peptide.

33. A eukaryotic cell transfected with the vector of claim 32.

34. A eukaryotic cell in which the first and second fusion protein according to the method of any one of claims 4 to 6 is expressed, wherein the cell is transfected with
A. a vector comprising a nucleic acid molecule encoding the fusion protein comprising
(i) a fluorescent (poly)peptide;
(ii) a (poly)peptide that, when expressed in a cell, accumulates at distinct sites in the nucleus of the cell; and
(iii) a bait (poly)peptide; and
B. a vector comprising a nucleic acid molecule encoding the fusion protein comprising
(i) a fluorescent (poly)peptide, the excitation and/or emission wavelength of which differs from that of the fluorescent (poly)peptide comprised in the fusion protein encoded by the nucleic acid molecule according to A; and
(ii) a prey (poly)peptide.

35. A eukaryotic cell having multiple copies of the lac-operator stably integrated in its DNA and stably expressing a protein comprising (i) a (poly)peptide that binds to the lac-operator; and (ii) a (poly)peptide specifically binding to green fluorescent protein (GFP).

36. The method of any one of claims 1 to 3 and 7 to 31 or the eukaryotic cell of claim 35, wherein said (poly)peptide specifically binding to green fluorescent protein (GFP) comprises a (poly)peptide having the amino acid sequence of SEQ ID NO: 7 or 9.

## Patentansprüche

1. *In vitro* Verfahren zum Nachweis von Protein-Protein-Interaktionen, umfassend:
(a) Expression eines ersten Fusionsproteins umfassend
(i) ein (Poly)Peptid, das sich, wenn es in einer Zelle exprimiert wird, an bestimmten Stellen im Zellkern der Zelle ansammelt; und
(ii) ein (Poly)Peptid, das spezifisch an grün-fluoreszierendes Protein (GFP) bindet
in einer eukaryontischen Zelle
(b) Expression eines zweiten Fusionsproteins umfassend
(i) GFP; und
(ii) ein Köder ("Bait") - (Poly)Peptid
in derselben Zelle
(c) Expression eines dritten Fusionsproteins umfassend
(i) ein fluoreszierendes (Poly)Peptid, dessen Anregungs- und/oder EmissionsWellenlänge sich von der von GFP unterscheidet; und
(ii) ein Beute ("Prey") - (Poly)Peptid
in derselben Zelle
(d) Nachweis der Fluoreszenz-Emission der fluoreszierenden Teile des zweiten und des dritten Fusionsproteins in der Zelle nach Anregung,
wobei eine Ko-Lokalisierung der Fluoreszenz-Emission der beiden Fusionsproteine im Zellkern eine Interaktion von Köder ("Bait") - und Beute ("Prey") - (Poly)Peptid anzeigt.

2. *In vitro* Verfahren zur Identifizierung eines Stoffes, der die Interaktion von zwei (Poly)Peptiden moduliert, wobei
(a) ein erstes Fusionsprotein umfassend
(i) ein (Poly)Peptid, das sich, wenn es in einer Zelle exprimiert wird, an bestimmten Stellen im Zellkern der Zelle ansammelt; und
(ii) ein (Poly)Peptid, das spezifisch an grün-fluoreszierendes Protein (GFP) bindet
in einer eukaryontischen Zelle exprimiert wird;
(b) ein zweites Fusionsprotein umfassend
(i) GFP; und
(ii) ein Köder ("Bait") - (Poly)Peptid
in derselben Zelle exprimiert wird;
(c) ein drittes Fusionsprotein umfassend
(i) ein fluoreszierendes (Poly)Peptid, dessen Anregungs- und/oder EmissionsWellenlänge sich von der von GFP unterscheidet; und
(ii) ein Beute ("Prey") - (Poly)Peptid, das bekanntlich oder vermutlich mit dem Köder ("Bait") - (Poly)Peptid interagiert
in derselben Zelle exprimiert wird;
(d) die Zelle mit einem Test-Stoff in Kontakt gebracht wird; und
(e) die Fluoreszenz-Emission der fluoreszierenden Teile des zweiten und des dritten Fusionsproteins in der Zelle nach Anregung nachgewiesen wird,
wobei eine Veränderung in dem Ausmaß der Ko-Lokalisierung der Fluoreszenz-Emission der fluoreszierenden Teile des zweiten und des dritten Fusionsproteins im Zellkern im Vergleich zu dem beobachteten Ausmaß im Zellkern einer Referenz-Zelle, die nicht mit dem Test-Stoff kontaktiert wurde, anzeigt, dass der Stoff fähig ist, die Interaktion des Köder ("Bait") - und des Beute ("Prey") - (Poly)Peptids zu modulieren.

3. *In vitro* Verfahren zum Bestimmen der relativen Stärke der Interaktion von zwei Proteinen mit einem dritten Protein, umfassend
(a) Expression eines ersten Fusionsproteins umfassend
(i) ein (Poly)Peptid, das sich, wenn es in einer Zelle exprimiert wird, an bestimmten Stellen im Zellkern der Zelle ansammelt; und
(ii) ein (Poly)Peptid, das spezifisch an grün-fluoreszierendes Protein (GFP) bindet
in einer eukaryontischen Zelle
(b) Expression eines zweiten Fusionsproteins umfassend
(i) GFP; und
(ii) ein Köder ("Bait") - (Poly)Peptid
in derselben Zelle
(c) Expression eines dritten Fusionsproteins umfassend
(i) ein fluoreszierendes (Poly)Peptid, dessen Anregungs- und/oder EmissionsWellenlänge sich von der von GFP unterscheidet; und
(ii) ein erstes Beute ("Prey") - (Poly)Peptid
in derselben Zelle
(d) Expression eines vierten Fusionsproteins umfassend
(i) ein fluoreszierendes (Poly)Peptid, dessen Anregungs- und/oder EmissionsWellenlänge sich von der von GFP und von der des fluoreszierenden (Poly)Peptids des dritten Fusionsproteins unterscheidet; und
(ii) ein zweites Beute ("Prey") - (Poly)Peptid
in derselben Zelle
(e) Nachweis der Fluoreszenz-Emission der fluoreszierenden Teile des zweiten und des dritten Fusionsproteins in der Zelle nach Anregung,
wobei ein unterschiedliches Ausmaß der Ko-Lokalisierung der Fluoreszenz-Emission des zweiten und dritten Fusionsproteins im Vergleich zu dem des zweiten und vierten Fusionsproteins im Zellkern anzeigt, dass das erste und das zweite Beute ("Prey") - (Poly)Peptid eine unterschiedliche Binde-Stärke für das Köder ("Bait") - (Poly)Peptid haben.

4. *In vitro* Verfahren zum Nachweis von Protein-Protein-Interaktionen, umfassend:
(a) Expression eines ersten Fusionsproteins umfassend
(i) ein fluoreszierendes (Poly)Peptid;
(ii) ein (Poly)Peptid, das sich, wenn es in einer Zelle exprimiert wird, an bestimmten Stellen im Zellkern der Zelle ansammelt; und
(iii) ein Köder ("Bait") - (Poly)Peptid
in einer eukaryontischen Zelle;
(b) Expression eines zweiten Fusionsproteins umfassend
(i) ein fluoreszierendes (Poly)Peptid, dessen Anregungs- und/oder EmissionsWellenlänge sich von der unterscheidet, die das fluoreszierende (Poly)Peptid hat, das im ersten Fusionsprotein umfasst ist; und
(ii) ein Beute ("Prey") - (Poly)Peptid
in derselben Zelle;
(c) Nachweis der Fluoreszenz-Emission der fluoreszierenden Teile des ersten und des zweiten Fusionsproteins in der Zelle nach Anregung,
wobei eine Ko-Lokalisierung der Fluoreszenz-Emission der beiden Fusionsproteine im Zellkern eine Interaktion von Köder ("Bait") - und Beute ("Prey") - (Poly)Peptid anzeigt.

5. *In vitro* Verfahren zur Identifizierung eines Stoffes, der die Interaktion von zwei (Poly)Peptiden moduliert, wobei
(a) ein erstes Fusionsprotein umfassend
(i) ein fluoreszierendes (Poly)Peptid;
(ii) ein (Poly)Peptid, das sich, wenn es in einer Zelle exprimiert wird, an bestimmten Stellen im Zellkern der Zelle ansammelt; und
(iii) ein Köder ("Bait") - (Poly)Peptid
in einer eukaryontischen Zelle exprimiert wird;
(b) ein zweites Fusionsprotein umfassend
(i) ein fluoreszierendes (Poly)Peptid, dessen Anregungs- und/oder EmissionsWellenlänge sich von der unterscheidet, die das fluoreszierende (Poly)Peptid hat, das im ersten Fusionsprotein umfasst ist; und
(ii) ein Beute ("Prey") - (Poly)Peptid, das bekanntlich oder vermutlich mit dem Köder ("Bait") - (Poly)Peptid interagiert
in derselben Zelle exprimiert wird;
(c) die Zelle mit einem Test-Stoff in Kontakt gebracht wird; und
(d) die Fluoreszenz-Emission der fluoreszierenden Teile des ersten und des zweiten Fusionsproteins in der Zelle nach Anregung nachgewiesen wird,
wobei eine Veränderung in dem Ausmaß der Ko-Lokalisierung der Fluoreszenz-Emission der fluoreszierenden Teile der beiden Fusionsproteine im Zellkern im Vergleich zu dem beobachteten Ausmaß im Zellkern einer Referenz-Zelle, die nicht mit dem Test-Stoff kontaktiert wurde, anzeigt, dass der Stoff fähig ist, die Interaktion des Köder ("Bait") - und des Beute ("Prey") - (Poly)Peptids zu modulieren.

6. *In vitro* Verfahren zum Bestimmen der relativen Stärke der Interaktion von zwei Proteinen mit einem dritten Protein, umfassend
(a) Expression eines ersten Fusionsproteins umfassend
(i) ein fluoreszierendes (Poly)Peptid;
(ii) ein (Poly)Peptid, das sich, wenn es in einer Zelle exprimiert wird, an bestimmten Stellen im Zellkern der Zelle ansammelt; und
(iii) ein Köder ("Bait") - (Poly)Peptid
in einer eukaryontischen Zelle;
(b) Expression eines zweiten Fusionsproteins umfassend
(i) ein fluoreszierendes (Poly)Peptid, dessen Anregungs- und/oder EmissionsWellenlänge sich von der unterscheidet, die das fluoreszierende (Poly)Peptid hat, das im ersten Fusionsprotein umfasst ist; und
(ii) ein erstes Beute ("Prey") - (Poly)Peptid
in derselben Zelle
(c) Expression eines dritten Fusionsproteins umfassend
(i) ein fluoreszierendes (Poly)Peptid, dessen Anregungs- und/oder EmissionsWellenlänge sich von der unterscheidet, die das fluoreszierende (Poly)Peptid hat, das im ersten und zweiten Fusionsprotein umfasst ist; und
(ii) ein zweites Beute ("Prey") - (Poly)Peptid
in derselben Zelle
(d) Nachweis der Fluoreszenz-Emission der fluoreszierenden Teile des ersten, des zweiten und des dritten Fusionsproteins in der Zelle nach Anregung,
wobei ein unterschiedliches Ausmaß der Ko-Lokalisierung der Fluoreszenz-Emission des ersten und zweiten Fusionsproteins im Vergleich zu dem des ersten und dritten Fusionsproteins im Zellkern anzeigt, dass das erste und das zweite Beute ("Prey") - (Poly)Peptid eine unterschiedliche Binde-Stärke für das Köder ("Bait") - (Poly)Peptid haben.

7. Verfahren nach Anspruch 2 oder 5, wobei eine Verringerung der Ko-Lokalisierung der fluoreszierenden Teile der beiden Fusionsproteine im Zellkern im Vergleich zu dem beobachteten Ausmaß im Zellkern einer Referenz-Zelle, die nicht mit dem Test-Stoff kontaktiert wurde, anzeigt, dass der Stoff fähig ist, die Interaktion des Köder ("Bait") - und des Beute ("Prey") - (Poly)Peptids zu schwächen.

8. Verfahren nach Anspruch 2 oder 5, wobei eine Zunahme der Ko-Lokalisierung der fluoreszierenden Teile der beiden Fusionsproteine im Zellkern im Vergleich zu dem beobachteten Ausmaß im Zellkern einer Referenz-Zelle, die nicht mit dem Test-Stoff kontaktiert wurde, anzeigt, dass der Stoff fähig ist, die Interaktion des Köder ("Bait") - und des Beute ("Prey") - (Poly)Peptids herbeizuführen oder zu verstärken.

9. Verfahren nach Anspruch 4, wobei der Nachweis verwendet wird, um die Abhängigkeit von Protein-Protein-Interaktionen von zellulären Vorgängen zu untersuchen, und wobei das Verfahren des Weiteren umfasst (d1) Überwachen der Fluoreszenz-Emission der fluoreszierenden Teile des ersten und des zweiten Fusionsproteins in der Zelle im Zuge eines oder mehrerer Vorgänge in der Zelle; wobei eine Veränderung in dem Ausmaß der Ko-Lokalisierung der Fluoreszenz-Emission der fluoreszierenden Teile der beiden Fusionsproteine in der Zelle anzeigt, dass die Interaktion von dem einen oder den mehreren zellulären Vorgängen abhängig ist.

10. Verfahren nach Anspruch 1, wobei der Nachweis verwendet wird, um die Abhängigkeit von Protein-Protein-Interaktionen von zellulären Vorgängen zu untersuchen, und wobei das Verfahren des Weiteren umfasst (d1) Überwachen der Fluoreszenz-Emission der fluoreszierenden Teile des zweiten und des dritten Fusionsproteins in der Zelle im Zuge eines oder mehrerer Vorgänge in der Zelle; wobei eine Veränderung in dem Ausmaß der Ko-Lokalisierung der Fluoreszenz-Emission der fluoreszierenden Teile der beiden Fusionsproteine in der Zelle anzeigt, dass die Interaktion von dem einen oder den mehreren zellulären Vorgängen abhängig ist.

11. Verfahren nach Anspruch 9 oder 10, wobei der zelluläre Vorgang der Zellzyklus, Sekretion, Translokation oder Signaltransduktion ist.

12. Verfahren nach Anspruch 4, wobei der Nachweis verwendet wird, um die Stärke einer Protein-Protein-Interaktion zu bestimmen, und wobei das Verfahren dann, wenn eine Ko-Lokalisierung der Fluoreszenz-Emission der fluoreszierenden Teile der beiden Fusionsproteine nachgewiesen ist, des Weiteren umfasst (d2) selektive Löschung der Fluoreszenz des zweiten Fusionsproteins und Überwachen der Wiederherstellung der Ko-Lokalisierung der Fluoreszenz-Emission der fluoreszierenden Teile der beiden Fusionsproteine mit der Zeit, wobei die Zeit, die notwendig ist, um die Ko-Lokalisierung herzustellen, anzeigt, wie stark die Protein-Protein-Interaktion ist.

13. Verfahren nach Anspruch 1, wobei der Nachweis verwendet wird, um die Stärke einer Protein-Protein-Interaktion zu bestimmen, und wobei das Verfahren dann, wenn eine Ko-Lokalisierung der Fluoreszenz-Emission der fluoreszierenden Teile der beiden Fusionsproteine nachgewiesen ist, des Weiteren umfasst (d2) selektive Löschung der Fluoreszenz des dritten Fusionsproteins und Überwachen der Wiederherstellung der Ko-Lokalisierung der Fluoreszenz-Emission der fluoreszierenden Teile der beiden Fusionsproteine mit der Zeit, wobei die Zeit, die notwendig ist, um die Ko-Lokalisierung herzustellen, anzeigt, wie stark die Protein-Protein-Interaktion ist.

14. Verfahren nach einem der Ansprüche 1 bis 13, wobei das (Poly)Peptid, das sich an bestimmten Stellen im Zellkern der Zelle ansammelt, wenn es in einer Zelle exprimiert wird, direkt mit proteinösen oder nicht-proteinösen Strukturen interagiert, die sich an bestimmten Stellen im Zellkern der Zelle angesammelt haben.

15. Verfahren nach einem der Ansprüche 1 bis 13, wobei das (Poly)Peptid, das sich an bestimmten Stellen im Zellkern der Zelle ansammelt, wenn es in einer Zelle exprimiert wird, indirekt mit proteinösen oder nicht-proteinösen Strukturen interagiert, die sich an bestimmten Stellen im Zellkern der Zelle angesammelt haben.

16. Verfahren nach einem der Ansprüche 1 bis 13, wobei die Elemente (i), (ii) und/oder (iii) des ersten Fusionsproteins und/oder die Elemente (i) und (ii) des zweiten Fusionsproteins und/oder die Elemente (i) und (ii) des dritten Fusionsproteins und/oder die Elemente (i) und (ii) des vierten Fusionsproteins mittels eines Linkers verbunden sind.

17. Verfahren nach einem der Ansprüche 1 bis 16, wobei eines oder mehrere der Beute ("Prey") - (Poly)Peptide ein Kernlokalisierungssignal ("nuclear localisation signal") umfasst.

18. Verfahren nach einem der Ansprüche 1 bis 3, wobei das zweite und/oder dritte und/oder vierte Fusionsprotein ein Kernlokalisierungssignal ("nuclear localisation signal") umfasst.

19. Verfahren nach einem der Ansprüche 1 bis 18, wobei die Expression in der eukaryontischen Zelle dadurch bewirkt wird, dass Nukleinsäuremoleküle, die das erste und zweite Fusionsprotein kodieren, in einem oder mehreren Vektoren transfiziert werden.

20. Verfahren nach einem der Ansprüche 1 bis 19, wobei das (Poly)Peptid, das sich an bestimmten Stellen im Zellkern der Zelle ansammelt, in die Zelle eingeführt worden ist.

21. Verfahren nach einem der Ansprüche 1 bis 20, wobei die bestimmten Stellen der Zellen inerte Strukturen bilden.

22. Verfahren nach Anspruch 21, wobei die inerte Struktur Zellkern-Lamina oder Zellkern-Flecken ("Speckles") ist/sind.

23. Verfahren nach einem der Ansprüche 1 bis 20, wobei das (Poly)Peptid, das sich an bestimmten Stellen im Zellkern der Zelle ansammelt, wenn es in einer Zelle exprimiert wird, mit PML-Körpern ("PML-Bodies") interagiert.

24. Verfahren nach einem der Ansprüche 1 bis 23, wobei das (Poly)Peptid, das sich an bestimmten Stellen im Zellkern ansammelt, für die Zelle heterolog ist.

25. Verfahren nach einem der Ansprüche 1 bis 21 und 24, wobei das (Poly)Peptid, das sich an bestimmten Stellen im Zellkern der Zelle ansammelt, wenn es in einer Zelle exprimiert wird, mit DNA interagiert.

26. Verfahren nach Anspruch 25, wobei die DNA der Lac-Operator ist.

27. Verfahren nach Anspruch 26, wobei der Lac-Operator in mehreren Kopien im Zellkern vorhanden ist.

28. Verfahren nach einem der Ansprüche 1 bis 27, wobei das erste Fusionsprotein Lacl umfasst.

29. Verfahren nach einem der Ansprüche 1 bis 28, wobei der Nachweis unter Verwendung eines Fluoreszenzmikroskops durchgeführt wird.

30. Verfahren nach einem der Ansprüche 1 bis 29, wobei die eukaryontische Zelle eine lebende Zelle ist.

31. Verfahren nach einem der Ansprüche 1 bis 30, des Weiteren umfassend das Lokalisieren des Köder ("Bait") - und/oder Beute ("Prey") - (Poly)Peptids in der Zelle.

32. Vektor zur Verwendung in der Expression des ersten und zweiten Fusionsproteins gemäß des Verfahrens nach einem der Ansprüche 4 bis 6 in einer eukaryontischen Zelle, umfassend
A. ein Nukleinsäuremolekül, das das Fusionsprotein kodiert, das
(i) ein fluoreszierendes (Poly)Peptid;
(ii) ein (Poly)Peptid, das sich, wenn es in einer Zelle exprimiert wird, an bestimmten Stellen im Zellkern der Zelle ansammelt; und
(iii) ein Köder ("Bait") - (Poly)Peptid
umfasst; und
B. ein Nukleinsäuremolekül, das das Fusionsprotein kodiert, das
(i) ein fluoreszierendes (Poly)Peptid, dessen Anregungs- und/oder EmissionsWellenlänge sich von der unterscheidet, die das fluoreszierende (Poly)Peptid hat, das in dem Fusionsprotein umfasst ist, das durch das Nukleinsäuremolekül gemäß A kodiert wird; und
(ii) ein Beute ("Prey") - (Poly)Peptid
umfasst.

33. Eukaryontische Zelle, die mit dem Vektor nach Anspruch 32 transfiziert wurde.

34. Eukaryontische Zelle, in der das erste und zweite Fusionsprotein gemäß des Verfahrens nach einem der Ansprüche 4 bis 6 exprimiert wird, wobei die Zelle transfiziert ist mit
A. einem Vektor, der ein Nukleinsäuremolekül umfasst, das das Fusionsprotein kodiert, das
(i) ein fluoreszierendes (Poly)Peptid;
(ii) ein (Poly)Peptid, das sich, wenn es in einer Zelle exprimiert wird, an bestimmten Stellen im Zellkern der Zelle ansammelt; und
(iii) ein Köder ("Bait") - (Poly)Peptid
umfasst; und
B. einem Vektor, der ein Nukleinsäuremolekül umfasst, das das Fusionsprotein kodiert, das
(i) ein fluoreszierendes (Poly)Peptid, dessen Anregungs- und/oder EmissionsWellenlänge sich von der unterscheidet, die das fluoreszierende (Poly)Peptid hat, das in dem Fusionsprotein umfasst ist, das durch das Nukleinsäuremolekül gemäß A kodiert wird; und
(ii) ein Beute ("Prey") - (Poly)Peptid
umfasst.

35. Eukaryontische Zelle, die mehrere stabil in ihre DNA integrierte Kopien des Lac-Operators hat und die ein Protein umfassend (i) ein (Poly)Peptid, das an den Lac-Operator bindet und (ii) ein (Poly)Peptid, das spezifisch an grün-fluoreszierendes Protein (GFP) bindet, stabil exprimiert.

36. Verfahren nach einem der Ansprüche 1 bis 3 und 7 bis 31, oder eukaryontische Zelle nach Anspruch 35, wobei das (Poly)Peptid, das spezifisch an grün-fluoreszierendes Protein (GFP) bindet, ein (Poly)Peptid umfasst, dass die Aminosäuresequenz von SEQ ID NO:7 oder 9 hat.

## Revendications

1. Procédé in vitro de détection des interactions protéine-protéine comprenant :
(a) l'expression dans une cellule eucaryote d'une première protéine de fusion comprenant :
(i) un (poly)peptide qui, quand il est exprimé dans une cellule, s'accumule en des sites distincts dans le noyau de la cellule ; et
(ii) un (poly)peptide se liant spécifiquement à la protéine fluorescente verte (GFP)
(b) l'expression dans la même cellule d'une deuxième protéine de fusion comprenant
(i) la GFP ; et
(ii) un (poly)peptide-appât
(c) l'expression dans la même cellule d'une troisième protéine de fusion comprenant
(i) un (poly)peptide fluorescent, dont la longueur d'onde d'excitation et/ou d'émission diffère de celle de la GFP ; et
(ii) un (poly)peptide-proie
(d) la détection de l'émission de fluorescence des parties fluorescentes des deuxième et troisième protéines de fusion dans la cellule après excitation,
dans lequel une co-localisation de l'émission de fluorescence des deux protéines de fusion dans le noyau de la cellule indique une interaction des (poly)peptides appât et proie.

2. Procédé in vitro d'identification d'un composé modulant l'interaction de deux (poly)peptides
(a) exprimant dans une cellule eucaryote une première protéine de fusion comprenant
(i) un (poly)peptide qui, quand il est exprimé dans une cellule, s'accumule en des sites distincts dans le noyau de la cellule ; et
(ii) un (poly)peptide se liant spécifiquement à la protéine fluorescente verte (GFP)
(b) exprimant dans la même cellule une deuxième protéine de fusion comprenant
(i) la GFP ; et
(ii) un (poly)peptide-appât
(c) exprimant dans la même cellule une troisième protéine de fusion comprenant
(i) un (poly)peptide fluorescent, dont la longueur d'onde d'excitation et/ou d'émission diffère de celle de la GFP ; et
(ii) un (poly)peptide-proie connu pour, ou suspecté d'interagir avec le (poly)peptide-appât,
(d) la mise en contact de la cellule avec un composé test ; et
(e) la détection de l'émission de fluorescence des parties fluorescentes des deuxième et troisième protéines de fusion dans la cellule après excitation ;
dans lequel une modification du degré de co-localisation de l'émission de fluorescence des parties fluorescentes des deuxième et troisième protéines de fusion dans le noyau de la cellule, comparativement à celui observé dans le noyau d'une cellule de référence qui n'a pas été mise en contact avec le composé test, indique que le composé est capable de moduler l'interaction des (poly)peptides appât et proie.

3. Procédé in vitro de détermination de la force relative de l'interaction de deux protéines avec une troisième protéine comprenant :
(a) l'expression dans une cellule eucaryote d'une première protéine de fusion comprenant
(i) un (poly)peptide qui, quand il est exprimé dans une cellule, s'accumule en des sites distincts dans le noyau de la cellule ; et
(ii) un (poly)peptide se liant spécifiquement à la protéine fluorescente verte (GFP)
(b) l'expression dans la même cellule d'une deuxième protéine de fusion comprenant
(i) la GFP ; et
(ii) un (poly)peptide-appât
(c) l'expression dans la même cellule d'une troisième protéine de fusion comprenant
(i) un (poly)peptide fluorescent, dont la longueur d'onde d'excitation et/ou d'émission diffère de celle de la GFP ; et
(ii) un premier (poly)peptide-proie
(d) l'expression dans la même cellule d'une quatrième protéine de fusion comprenant
(i) un (poly)peptide fluorescent, dont la longueur d'onde d'excitation et/ou d'émission diffère de celle de la GFP et de celle du (poly)peptide fluorescent de ladite troisième protéine de fusion ; et
(ii) un second (poly)peptide-proie
(e) la détection de l'émission de fluorescence des parties fluorescentes des deuxième et troisième protéines de fusion dans la cellule après excitation,
dans lequel toute extension de la co-localisation de l'émission de fluorescence des deuxième et troisième protéines de fusion différente, comparativement à celle des deuxième et quatrième protéines de fusion dans le noyau de la cellule, indique une force de liaison différente des premier et second (poly)peptides-proie au (poly)peptide-appât.

4. Procédé in vitro de détection des interactions protéine-protéine comprenant :
(a) l'expression dans une cellule eucaryote d'une première protéine de fusion comprenant
(i) un (poly)peptide fluorescent ;
(ii) un (poly)peptide qui, quand il est exprimé dans une cellule, s'accumule en des sites distincts dans le noyau de la cellule ; et
(iii) un (poly)peptide-appât ;
(b) l'expression dans la même cellule d'une seconde protéine de fusion comprenant
(i) un (poly)peptide fluorescent, dont la longueur d'onde d'excitation et/ou d'émission diffère de celle du (poly)peptide fluorescent compris dans ladite première protéine de fusion ; et
(ii) un (poly)peptide-proie ;
(c) la détection de l'émission de fluorescence des parties fluorescentes des première et seconde protéines de fusion dans la cellule après excitation,
dans lequel une co-localisation de l'émission de fluorescence des deux protéines de fusion dans le noyau de la cellule indique une interaction des (poly)peptides appât et proie.

5. Procédé in vitro d'identification d'un composé modulant l'interaction de deux (poly)peptides
(a) exprimant dans une cellule eucaryote une première protéine de fusion comprenant
(i) un (poly)peptide fluorescent
(ii) un (poly)peptide qui, quand il est exprimé dans une cellule, s'accumule en des sites distincts dans le noyau de la cellule ; et
(iii) un (poly)peptide-appât
(b) exprimant dans la même cellule une seconde protéine de fusion comprenant
(i) un (poly)peptide fluorescent, dont la longueur d'onde d'excitation et/ou d'émission diffère de celle du (poly)peptide fluorescent compris dans ladite première protéine de fusion ; et
(ii) un (poly)peptide-proie connu pour, ou suspecté d'interagir avec le (poly)peptide-appât,
(c) la mise en contact de la cellule avec un composé test ; et
(d) la détection de l'émission de fluorescence des parties fluorescentes des première et seconde protéines de fusion dans la cellule après excitation,
dans lequel une modification du degré de co-localisation de l'émission de fluorescence des parties fluorescentes des deux protéines de fusion dans le noyau de la cellule, comparativement à celui observé dans le noyau d'une cellule de référence qui n'a pas été mise en contact avec le composé test, indique que le composé est capable de moduler l'interaction des (poly)peptides appât et proie.

6. Procédé in vitro de détermination de la force relative de l'interaction de deux protéines avec une troisième protéine comprenant
(a) l'expression dans une cellule eucaryote d'une première protéine de fusion comprenant
(i) un (poly)peptide fluorescent ;
(ii) un (poly)peptide qui, quand il est exprimé dans une cellule, s'accumule en des sites distincts dans le noyau de la cellule ; et
(iii) un (poly)peptide-appât
(b) l'expression dans la même cellule d'une deuxième protéine de fusion comprenant
(i) un (poly)peptide fluorescent, dont la longueur d'onde d'excitation et/ou d'émission diffère de celle du (poly)peptide fluorescent compris dans ladite première protéine de fusion ; et
(ii) un premier (poly)peptide-proie
(c) l'expression dans la même cellule d'une troisième protéine de fusion comprenant
(i) un (poly)peptide fluorescent, dont la longueur d'onde d'excitation et/ou d'émission diffère de celle du (poly)peptide fluorescent compris dans lesdites première et deuxième protéines de fusion ; et
(ii) un second (poly)peptide-proie
(d) la détection de l'émission de fluorescence des parties fluorescentes des première, deuxième et troisième protéines de fusion dans la cellule après excitation,
dans lequel toute extension de la co-localisation de l'émission de fluorescence des première et deuxième protéines de fusion différente, comparativement à celle des première et troisième protéines de fusion dans le noyau de la cellule, indique une force de liaison différente des première et deuxième protéines-proies au (poly)peptide-appât.

7. Procédé selon la revendication 2 ou 5, dans lequel une diminution de la colocalisation des parties fluorescentes des deux protéines de fusion dans le noyau de la cellule, comparativement à celle observée dans le noyau d'une cellule de référence qui n'a pas été mise en contact avec le composé test, indique que le composé est capable d'affaiblir l'interaction des (poly)peptides-appât et proie.

8. Procédé selon la revendication 2 ou 5, dans lequel un accroissement de la colocalisation des parties fluorescentes des deux protéines de fusion dans le noyau de la cellule, comparativement à celle observée dans le noyau d'une cellule de référence qui n'a pas été mise en contact avec le composé test, indique que le composé est capable d'induire ou de renforcer l'interaction des (poly)peptides-appât et proie.

9. Procédé selon la revendication 4, dans lequel la détection est utilisée pour étudier la dépendance des interactions protéine-protéine vis-à-vis de processus cellulaires et dans lequel le procédé comprend en outre (d1) la surveillance de l'émission de fluorescence des parties fluorescentes des première et seconde protéines de fusion dans la cellule au cours d'un ou de plusieurs processus cellulaires ; dans lequel une modification du degré de co-localisation de l'émission de fluorescence des parties fluorescentes des deux protéines de fusion dans la cellule indique une dépendance de l'interaction vis-à-vis dudit ou desdits processus cellulaires.

10. Procédé selon la revendication 1, dans lequel la détection est utilisée pour étudier la dépendance des interactions protéine-protéine vis-à-vis de processus cellulaires et dans lequel le procédé comprend en outre (d1) la surveillance de l'émission de fluorescence des parties fluorescentes des deuxième et troisième protéines de fusion dans la cellule au cours d'un ou de plusieurs processus cellulaires ; dans lequel une modification du degré de co-localisation de l'émission de fluorescence des parties fluorescentes des deux protéines de fusion dans la cellule indique une dépendance de l'interaction vis-à-vis dudit ou desdits processus cellulaires.

11. Procédé selon la revendication 9 ou 10, dans lequel le processus cellulaire est le cycle cellulaire, la sécrétion, la translocation ou la transduction d'un signal.

12. Procédé selon la revendication 4, dans lequel la détection est utilisée pour étudier la force d'une interaction protéine-protéine et dans lequel le procédé comprend en outre dans le cas où une co-localisation de l'émission de fluorescence des parties fluorescentes des deux protéines de fusion est détectée (d2) l'extinction sélective de la fluorescence de ladite deuxième protéine de fusion et la surveillance de la restauration de la co-localisation de l'émission de fluorescence des parties fluorescentes des deux protéines de fusion dans le temps, dans lequel le temps nécessaire à l'établissement de la co-localisation indique la force de l'interaction protéine-protéine.

13. Procédé selon la revendication 1, dans lequel la détection est utilisée pour déterminer la force d'une interaction protéine-protéine et dans lequel la procédé comprend en outre dans le cas où une co-localisation de l'émission de fluorescence des parties fluorescentes des deux protéines de fusion est détectée (d2) l'extinction sélective de la fluorescence de ladite troisième protéine de fusion et la surveillance de la restauration de la co-localisation de l'émission de fluorescence des parties fluorescentes des deux protéines de fusion dans le temps, le temps nécessaire à l'établissement de la co-localisation indiquant la force de l'interaction protéine-protéine.

14. Procédé selon l'une quelconque des revendications 1 à 13 dans lequel le (poly)peptide qui, quand il est exprimé dans une cellule, s'accumule en des sites distincts dans le noyau de la cellule, interagit directement avec les structures protéiques et non protéiques qui se sont accumulées en des sites distincts dans le noyau de la cellule.

15. Procédé selon l'une quelconque des revendications 1 à 13 dans lequel le (poly)peptide qui, quand il est exprimé dans une cellule, s'accumule en des sites distincts dans le noyau de la cellule, interagit indirectement avec les structures protéiques et non protéiques qui se sont accumulées en des sites distincts dans le noyau de la cellule.

16. Procédé selon l'une quelconque des revendications 1 à 13, dans lequel les composants (i), (ii) et/ou (iii) de ladite première protéine de fusion et/ou les composants (i) et (ii) de ladite deuxième protéine de fusion et/ou les composants (i) et (ii) de ladite troisième protéine de fusion et/ou les composants (i) et (ii) de ladite quatrième protéine de fusion sont reliés par l'intermédiaire d'un lieur.

17. Procédé selon l'une quelconque des revendications 1 à 16, dans lequel l'un quelconque des (poly)peptides-proies comprend un signal de localisation nucléaire.

18. Procédé selon l'une quelconque des revendications 1 à 3, dans lequel ladite deuxième et/ou troisième et/ou quatrième protéine de fusion comprend un signal de localisation nucléaire.

19. Procédé selon l'une quelconque des revendications 1 à 18, dans lequel l'expression dans la cellule eucaryote est mise en oeuvre par transfection des molécules d'acides nucléiques codant pour lesdites première et deuxième protéines de fusion dans un ou plusieurs vecteurs.

20. Procédé selon l'une quelconque des revendications 1 à 19, dans lequel le (poly)peptide qui s'est accumulé en des sites distincts du noyau de la cellule a été introduit dans la cellule.

21. Procédé selon l'une quelconque des revendications 1 à 20, dans lequel les sites distincts de la cellule forment des structures inertes.

22. Procédé selon la revendication 21, dans lequel la structure interne est la lamina nucléaire ou des tâches nucléaires.

23. Procédé selon l'une quelconque des revendications 1 à 20, dans lequel le (poly)peptide qui, quand il est exprimé dans une cellule, s'accumule en des sites distincts dans le noyau de la cellule, interagit avec des corps PML.

24. Procédé selon l'une quelconque des revendications 1 à 23, dans lequel le (poly)peptide qui s'est accumulé en des sites distincts du noyau est hétérologue à la cellule.

25. Procédé selon l'une quelconque des revendications 1 à 21 et 24, dans lequel le (poly)peptide qui, quand il est exprimé dans une cellule, s'accumule en des sites distincts dans le noyau de la cellule interagit avec l'ADN.

26. Procédé selon la revendication 25, dans lequel l'ADN est l'opérateur lac.

27. Procédé selon la revendication 26, dans lequel l'opérateur lac est présent dans le noyau en de multiples copies.

28. Procédé selon l'une quelconque des revendications 1 à 27, dans lequel ladite première protéine de fusion comprend Lacl.

29. Procédé selon l'une quelconque des revendications 1 à 28, dans lequel la détection est mise en oeuvre à l'aide d'un microscope à fluorescence.

30. Procédé selon l'une quelconque des revendications 1 à 29, dans lequel la cellule eucaryote est une cellule vivante.

31. Procédé selon l'une quelconque des revendications 1 à 30 comprenant en outre la localisation des (poly)peptides-appât et/ou proie dans la cellule.

32. Vecteur pouvant être utilisé pour exprimer la première et la seconde protéine de fusion selon le procédé décrit dans l'une quelconque des revendications 4 à 16 dans une cellule eucaryote comprenant
A. une molécule d'acide nucléique codant pour la protéine de fusion comprenant
(i) un (poly)peptide fluorescent ;
(ii) un (poly)peptide qui, quand il est exprimé dans une cellule, s'accumule en des sites distincts dans le noyau de la cellule ; et
(iii) un (poly)peptide-appât ; et
B. une molécule d'acide nucléique codant pour la protéine de fusion comprenant
(i) un (poly)peptide fluorescent, dont la longueur d'onde d'excitation et/ou d'émission diffère de celle du (poly)peptide fluorescent compris dans la protéine de fusion codée par la molécule d'acide nucléique selon A ; et
(ii) un (poly)peptide-proie.

33. Cellule eucaryote transfectée par le vecteur selon la revendication 32.

34. Cellule eucaryote dans laquelle les première et seconde protéines de fusion selon la procédé décrite dans l'une quelconque des revendications 4 à 6 sont exprimées, dans laquelle la cellule est transfectée avec
A. un vecteur comprenant une molécule d'acide nucléique codant pour la protéine de fusion comprenant
(i) un (poly)peptide fluorescent ;
(ii) un (poly)peptide qui, quand il est exprimé dans une cellule, s'accumule en des sites distincts dans le noyau de la cellule ; et
(iii) un (poly)peptide-appât ; et
B. un vecteur comprenant une molécule d'acide nucléique codant pour la protéine de fusion comprenant
(i) un (poly)peptide fluorescent, dont la longueur d'onde d'excitation et/ou d'émission diffère de celle du (poly)peptide fluorescent compris dans la protéine de fusion codée par la molécule d'acide nucléique selon A ; et
(ii) un (poly)peptide-proie.

35. Cellule eucaryote comportant de multiples copies de l'opérateur lac intégré de manière stable dans son ADN et exprimant de manière stable une protéine comprenant (i) un (poly)peptide qui se lie à l'opérateur lac ; et (ii) un (poly)peptide se liant spécifiquement à la protéine fluorescente verte (GFP).

36. Procédé selon l'une quelconque des revendications 1 à 3 et 7 à 31 ou cellule eucaryote selon la revendication 35, dans lequel ledit (poly)peptide se liant spécifiquement à la protéine fluorescente verte (GFP) comprend un (poly)peptide ayant la séquence en acides aminés de la SEQ ID No : 7 ou 9.


### SEQUENCE LISTING

<110> Ludwig-Maximilians-Universitat München
<120> A fluorescent two-hybrid (F2H) assay for direct visualization of protein interactions in living cells
<130> N3214 EP
<160> 10
<170> PatentIn version 3.3
<210> 1
   <211> 238
   <212> PRT
   <213> Aequorea victoria
<400> 1
   Met Ser Lys Gly Glu Glu Leu Phe Thr Gly Val Val Pro Ile Leu Val
   1               5                   10                  15
   Glu Leu Asp Gly Asp Val Asn Gly His Lys Phe Ser Val Ser Gly Glu
               20                  25                  30
   Gly Glu Gly Asp Ala Thr Tyr Gly Lys Leu Thr Leu Lys Phe Ile Cys
           35                  40                  45
   Thr Thr Gly Lys Leu Pro Val Pro Trp Pro Thr Leu Val Thr Thr Phe
       50                  55                  60
   Ser Tyr Gly Val Gln Cys Phe Ser Arg Tyr Pro Asp His Met Lys Gln
   65                  70                  75                  80
   His Asp Phe Phe Lys Ser Ala Met Pro Glu Gly Tyr Val Gln Glu Arg
                   85                  90                  95
   Thr Ile Phe Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Met Ala Asp Lys Gln Lys Asn Gly
   145                 150                 155                 160
   Ile Lys Val Asn Phe Lys Ile Arg His Asn Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Lys
   225                 230                 235
<210> 2
   <211> 717
   <212> DNA
   <213> Aequorea victoria
<400> 2
   atgagtaaag gagaagaact tttcactgga gttgtcccaa ttcttgttga attagatggt      60
   gatgttaatg ggcacaaatt ttctgtcagt ggagagggtg aaggtgatgc aacatacgga     120
   aaacttaccc ttaaatttat ttgcactact ggaaaactac ctgttccatg gccaacactt     180
   gtcactactt tctcttatgg tgttcaatgc ttttcaagat acccagatca tatgaaacag     240
   catgactttt tcaagagtgc catgcccgaa ggttatgtac aggaaagaac tatatttttc     300
   aaagatgacg ggaactacaa gacacgtgct gaagtcaagt ttgaaggtga tacccttgtt     360
   aatagaatcg agttaaaagg tattgatttt aaagaagatg gaaacattct tggacacaaa     420
   ttggaataca actataactc acacaatgta tacatcatgg cagacaaaca aaagaatgga     480
   atcaaagtta acttcaaaat tagacacaac attgaagatg gaagcgttca actagcagac     540
   cattatcaac aaaatactcc aattggcgat ggccctgtcc ttttaccaga caaccattac     600
   ctgtccacac aatctgccct ttcgaaagat cccaacgaaa agagagacca catggtcctt     660
   cttgagtttg taacagctgc tgggattaca catggcatgg atgaactata caaataa        717
<210> 3
   <211> 225
   <212> PRT
   <213> Discosoma
<400> 3
   Met Arg Ser Ser Lys Asn Val Ile Lys Glu Phe Met Arg Phe Lys Val
   1               5                   10                  15
   Arg Met Glu Gly Thr Val Asn Gly His Glu Phe Glu Ile Glu Gly Glu
               20                  25                  30
   Gly Glu Gly Arg Pro Tyr Glu Gly His Asn Thr Val Lys Leu Lys Val
           35                  40                  45
   Thr Lys Gly Gly Pro Leu Pro Phe Ala Trp Asp Ile Leu Ser Pro Gln
       50                  55                  60
   Phe Gln Tyr Gly Ser Lys Val Tyr Val Lys His Pro Ala Asp Ile Pro
   65                  70                  75                  80
   Asp Tyr Lys Lys Leu Ser Phe Pro Glu Gly Phe Lys Trp Glu Arg Val
                   85                  90                  95
   Met Asn Phe Glu Asp Gly Gly Val Val Thr Val Thr Gln Asp Ser Ser
               100                 105                 110
   Leu Gln Asp Gly Cys Phe Ile Tyr Lys Val Lys Phe Ile Gly Val Asn
           115                 120                 125
   Phe Pro Ser Asp Gly Pro Val Met Gln Lys Lys Thr Met Gly Trp Glu
       130                 135                 140
   Ala Ser Thr Glu Arg Leu Tyr Pro Arg Asp Gly Val Leu Lys Gly Glu
   145                 150                 155                 160
   Ile His Lys Ala Leu Lys Leu Lys Asp Gly Gly His Tyr Leu Val Glu
                   165                 170                 175
   Phe Lys Ser Ile Tyr Met Ala Lys Lys Pro Val Gln Leu Pro Gly Tyr
               180                 185                 190
   Tyr Tyr Val Asp Ser Lys Leu Asp Ile Thr Ser His Asn Glu Asp Tyr
           195                 200                 205
   Thr Ile Val Glu Gln Tyr Glu Arg Thr Glu Gly Arg His His Leu Phe
       210                 215                 220
   Leu
   225
<210> 4
   <211> 678
   <212> DNA
   <213> Discosoma
<400> 4
   atgaggtctt ccaagaatgt tatcaaggag ttcatgaggt ttaaggttcg catggaagga     60
   acggtcaatg ggcacgagtt tgaaatagaa ggcgaaggag aggggaggcc atacgaaggc    120
   cacaataccg taaagcttaa ggtaaccaag gggggacctt tgccatttgc ttgggatatt    180
   ttgtcaccac aatttcagta tggaagcaag gtatatgtca agcaccctgc cgacatacca    240
   gactataaaa agctgtcatt tcctgaagga tttaaatggg aaagggtcat gaactttgaa    300
   gacggtggcg tcgttactgt aacccaggat tccagtttgc aggatggctg tttcatctac    360
   aaggtcaagt tcattggcgt gaactttcct tccgatggac ctgttatgca aaagaagaca    420
   atgggctggg aagccagcac tgagcgtttg tatcctcgtg atggcgtgtt gaaaggagag    480
   attcataagg ctctgaagct gaaagacggt ggtcattacc tagttgaatt caaaagtatt    540
   tacatggcaa agaagcctgt gcagctacca gggtactact atgttgactc caaactggat    600
   ataacaagcc acaacgaaga ctatacaatc gttgagcagt atgaaagaac cgagggacgc    660
   caccatctgt tcctttag                                                  678
<210> 5
   <211> 229
   <212> PRT
   <213> artificial sequence
<220>
   <221> source
   <223> /note="Description of artificial sequence: mRFP1"
<400> 5
   Met Ala Ser Ser Glu Asp Val Ile Lys Glu Phe Met Arg Phe Lys Val
   1               5                   10                  15
   Arg Met Glu Gly Ser Val Asn Gly His Glu Phe Glu Ile Glu Gly Glu
               20                  25                  30
   Gly Glu Gly Arg Pro Tyr Glu Gly Thr Gln Thr Ala Lys Leu Lys Val
           35                  40                  45
   Thr Lys Gly Gly Pro Leu Pro Phe Ala Trp Asp Ile Leu Ser Pro Gln
       50                  55                  60
   Phe Gln Tyr Gly Ser Lys Ala Tyr Val Lys His Pro Ala Asp Ile Pro
   65                  70                  75                  80
   Asp Tyr Leu Lys Leu Ser Phe Pro Glu Gly Phe Lys Trp Glu Arg Val
                   85                  90                  95
   Met Asn Phe Glu Asp Gly Gly Val Val Thr Val Thr Gln Asp Ser Ser
               100                 105                 110
   Leu Gln Asp Gly Glu Phe Ile Tyr Lys Val Lys Leu Arg Gly Thr Asn
           115                 120                 125
   Phe Pro Ser Asp Gly Pro Val Met Gln Lys Lys Thr Met Gly Trp Glu
       130                 135                 140
   Ala Ser Thr Glu Arg Met Tyr Pro Glu Asp Gly Ala Leu Lys Gly Glu
   145                 150                 155                 160
   Ile Lys Met Arg Leu Lys Leu Lys Asp Gly Gly His Tyr Asp Ala Glu
                   165                 170                 175
   Val Lys Thr Thr Tyr Met Ala Lys Lys Pro Val Gln Leu Pro Gly Ala
               180                 185                 190
   Tyr Lys Thr Asp Ile Lys Leu Asp Ile Thr Ser His Asn Glu Asp Tyr
           195                 200                 205
   Thr Ile Val Glu Gln Tyr Glu Arg Ala Glu Gly Arg His Ser Thr Gly
       210                 215                 220
   Ala Gly Leu Tyr Lys
   225
<210> 6
   <211> 690
   <212> DNA
   <213> artificial sequence
<220>
   <221> source
   <223> /note="Description of artificial sequence: mRFP1"
<400> 6
   atggcctcct ccgaggacgt catcaaggag ttcatgcgct tcaaggtgcg catggagggc     60
   tccgtgaacg gccacgagtt cgagatcgag ggcgagggcg agggccgccc ctacgagggc    120
   acccagaccg ccaagctgaa ggtgaccaag ggcggccccc tgcccttcgc ctgggacatc    180
   ctgtcccctc agttccagta cggctccaag gcctacgtga agcaccccgc cgacatcccc    240
   gactacttga agctgtcctt ccccgagggc ttcaagtggg agcgcgtgat gaacttcgag    300
   gacggcggcg tggtgaccgt gacccaggac tcctccctgc aggacggcga gttcatctac    360
   aaggtgaagc tgcgcggcac caacttcccc tccgacggcc ccgtaatgca gaagaagacc    420
   atgggctggg aggcctccac cgagcggatg taccccgagg acggcgccct gaagggcgag    480
   atcaagatga ggctgaagct gaaggacggc ggccactacg acgccgaggt caagaccacc    540
   tacatggcca agaagcccgt gcagctgccc ggcgcctaca agaccgacat caagctggac    600
   atcacctccc acaacgagga ctacaccatc gtggaacagt acgagcgcgc cgagggccgc    660
   cactccaccg gcgccggcct gtacaagtaa                                     690
<210> 7
   <211> 117
   <212> PRT
   <213> artificial sequence
<220>
   <221> source
   <223> /note="Description of artificial sequence: VHH fragment of fluorescent chromobody against GFP"
<400> 7
   Met Ala Asp Val Gln Leu Val Glu Ser Gly Gly Ala Leu Val Gln Pro
   1               5                   10                  15
   Gly Gly Ser Leu Arg Leu Ser Cys Ala Ala Ser Gly Phe Pro Val Asn
               20                  25                  30
   Arg Tyr Ser Met Arg Trp Tyr Arg Gln Ala Pro Gly Lys Glu Arg Glu
           35                  40                  45
   Trp Val Ala Gly Met Ser Ser Ala Gly Asp Arg Ser Ser Tyr Glu Asp
       50                  55                  60
   Ser Val Lys Gly Arg Phe Thr Ile Ser Arg Asp Asp Ala Arg Asn Thr
   65                  70                  75                  80
   Val Tyr Leu Gln Met Asn Ser Leu Lys Pro Glu Asp Thr Ala Val Tyr
                   85                  90                  95
   Tyr Cys Asn Val Asn Val Gly Phe Glu Tyr Trp Gly Gln Gly Thr Gln
               100                 105                 110
   Val Thr Val Ser Ser
           115
<210> 8
   <211> 351
   <212> DNA
   <213> artificial sequence
<220>
   <221> source
   <223> /note="Description of artificial sequence: sequence encoding VHH fragment of fluorescent chromobody of SEQ ID NO: 7"
<400> 8
   atggccgatg tgcagctggt ggagtctggg ggagccttgg tgcagccggg ggggtctctg      60
   agactctcct gtgcagcctc tggattcccc gtcaatcgct atagtatgag gtggtaccgc     120
   caggctccag ggaaggagcg cgagtgggtc gcgggtatga gtagtgctgg tgatcgttca     180
   agttatgaag actccgtgaa gggccgattc accatctcca gagacgacgc caggaatacg     240
   gtgtatctgc aaatgaacag cctgaaacct gaggacacgg ccgtgtatta ctgtaatgtc     300
   aatgtgggct ttgagtactg gggccagggg acccaggtca ccgtctcctc a              351
<210> 9
   <211> 117
   <212> PRT
   <213> artificial sequence
<220>
   <221> source
   <223> /note="Description of artificial sequence: VHH fragment of fluorescent chromobody against GFP (mutant C92S)"
<400> 9
   Met Ala Asp Val Gln Leu Val Glu Ser Gly Gly Ala Leu Val Gln Pro
   1               5                   10                  15
   Gly Gly Ser Leu Arg Leu Ser Cys Ala Ala Ser Gly Phe Pro Val Asn
               20                  25                  30
   Arg Tyr Ser Met Arg Trp Tyr Arg Gln Ala Pro Gly Lys Glu Arg Glu
           35                  40                  45
   Trp Val Ala Gly Met Ser Ser Ala Gly Asp Arg Ser Ser Tyr Glu Asp
       50                  55                  60
   Ser Val Lys Gly Arg Phe Thr Ile Ser Arg Asp Asp Ala Arg Asn Thr
   65                  70                  75                  80
   Val Tyr Leu Gln Met Asn Ser Leu Lys Pro Glu Asp Thr Ala Val Tyr
                   85                  90                  95
   Tyr Ser Asn Val Asn Val Gly Phe Glu Tyr Trp Gly Gln Gly Thr Gln
               100                 105                 110
   Val Thr Val Ser Ser
           115
<210> 10
   <211> 351
   <212> DNA
   <213> artificial sequence
<220>
   <221> source
   <223> /note="Description of artificial sequence: sequence encoding VHH fragment of fluorescent chromobody of SEQ ID NO: 9"
<400> 10
   atggccgatg tgcagctggt ggagtctggg ggagccttgg tgcagccggg ggggtctctg      60
   agactctcct gtgcagcctc tggattcccc gtcaatcgct atagtatgag gtggtaccgc     120
   caggctccag ggaaggagcg cgagtgggtc gcgggtatga gtagtgctgg tgatcgttca     180
   agttatgaag actccgtgaa gggccgattc accatctcca gagacgacgc caggaatacg     240
   gtgtatctgc aaatgaacag cctgaaacct gaggacacgg ccgtgtatta ctcgaatgtc     300
   aatgtgggct ttgagtactg gggccagggg acccaggtca ccgtctcctc a            351